(19) Europäisches Patentamt
European Patent Office
Office européen des brevets

(11) Numéro de publication: **0 335 806 B1**

# (12) FASCICULE DE BREVET EUROPEEN

(45) Date de publication du fascicule du brevet: **02.11.95**

(51) Int. Cl.[6]: **C07D 213/57**, C07F 7/08, A01N 43/40, A01N 55/00

(21) Numéro de dépôt: **89420103.7**

(22) Date de dépôt: **23.03.89**

Le dossier contient des informations techniques présentées postérieurement au dépôt de la demande et ne figurant pas dans le présent fascicule.

## (54) Composés beta-phénoxynitrile.

(30) Priorité: **29.03.88 FR 8804411**

(43) Date de publication de la demande: **04.10.89 Bulletin 89/40**

(45) Mention de la délivrance du brevet: **02.11.95 Bulletin 95/44**

(84) Etats contractants désignés: **AT BE CH DE ES FR GB GR IT LI LU NL SE**

(56) Documents cités:
**EP-A- 0 145 620**
**EP-A- 0 164 088**
**EP-A- 0 214 793**
**EP-A- 0 311 051**
**GB-A- 1 175 693**

(73) Titulaire: **RHONE-POULENC AGROCHIMIE**
**14-20, rue Pierre Baizet**
**F-69009 Lyon (FR)**

(72) Inventeur: **Gadras, Alain**
**39, rue Louis Bouquet**
**F-69009 Lyon (FR)**
Inventeur: **Pepin, Régis**
**27 Montée Castellane**
**F-69140 Rillieux la Pape (FR)**

(74) Mandataire: **Brachotte, Charles et al**
**RHONE-POULENC AGROCHIMIE**
**Département Propriété Industrielle**
**B.P. 9163**
**F-69263 Lyon Cedex 09 (FR)**

EP 0 335 806 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux composés, à usage phytosanitaire, à groupements 3-pyridinyl $\beta$-phenoxy ou phenylthio nitrile. Elle concerne également les procédés de préparation desdits composés et les produits éventuellement utilisables à titre d'intermédiaires dans les procédés de préparation. Elle concerne ensuite l'utilisation à titre de fongicides de ces composés les compositions fongicides à base de ces composés et les procédés pour lutter contre les maladies fongiques des cultures utilisant ces composés. Elle concerne également un produit de multiplication des plantes cultivées qui a subi un traitement de protection par un composé de l'invention.

De nombreux produits à groupes pyridinyl, notamment des fongicides, sont déjà connus. En particulier par la demande de brevet EP-A-145620 on connait des fongicides dérivés de l'acide 2-(3-pyridyl) 2-phénylamino acétique. Par la demande de brevet EP-A-214793, on connait des fongicides, dérivés des 2-aryl 3-pyridyl propionitriles, par EP 164 088, des azolylnitriles fongicides et par GB 1175 693, des dérivés de pyridine fongicides.

Un but de la présente invention est de proposer d'autres composés fongicides à large spectre utiles notamment dans le traitement des maladies du pied comme le pietin verse ou de la feuille comme l'oîdium, la septoriose, la pyriculariose, les fusarioses, la rhynchosporiose, des maladies provoquées par les champignons pathogènes tels que Botrytis, Phoma, Aschochyta, Alternaria dans des cultures aussi diverses que les céréales, la vigne, le riz, le maîs, le soja par exemple.

L'invention concerne donc en premier lieu les composés de formule (I) indiquée en fin de description dans laquelle :

A représente une chaîne alkylène linéaire comportant au plus six atomes de carbone par exemple diméthylène, méthylène ou ramifiée comportant au plus six atomes de carbone à condition que la chaine linéaire soit différente de méthylène, par exemple éthyl éthylène.

R représente :

- un radical alkyle inférieur éventuellement substitué (notamment par un ou plusieurs substituants choisis parmi les atomes d'halogènes et les radicaux alcoxy inférieur, alkylthio inférieur, -Si $(CH_2)_x$ $R_1$ $R_2$ $R_3$, x = 0, 1, $R_1$, $R_2$, $R_3$ identiques ou différents étant alkyle inférieur, alcoxy inférieur, aryle (notamment phényle), aralkyle (notamment benzyle) ;
- un radical cycloalkyle comportant 3 à 7 chaînons (notamment cyclopropyle, cyclobutyle, cyclopentyle, cyclohexyle) lui-même éventuellement substitué de la même manière que le radical alkyle ;
- un radical aralkyle (notamment benzyle, phénéthyle) éventuellement substitué de la même manière que le radical alkyle ;
- un radical alcynyle ou alcényle inférieur mono ou poly-insaturé éventuellement substitué de la même manière que le groupement alkyle à condition qu'il n'y ait pas d'insaturation en alpha du carbone portant le groupe CN ;

  en outre, R peut être H si A est différent de méthylène.

X = O ou S

Y représente un atome d'halogène (notamment fluor, chlore, brome) un radical alkyle inférieur, un radical haloalkyle inférieur (notamment fluoroalkyle) un radical alcoxy inférieur, un radical haloalkoxy inférieur (notamment fluoroalkoxy), un radical alkylthio inférieur, un radical haloalkylthio inférieur (notamment fluoro alkylthio), un radical alkoxy carbonyle inférieur, un radical haloalkoxy carbonyle inférieur (notamment fluoroalkoxy carbonyle inférieur), un radical alcanoyloxy inférieur, un radical haloalcanoyloxy inférieur (notamment fluoroalcanoyloxy), un radical aryle (notamment phényle), un radical aralkyle (notamment benzyle), un radical cycloalkyle de 3 à 7 atomes de carbone, un radical cyano ou nitro ou hydroxyle.

n nombre entier positif ou nul, inférieur à 6 les groupements Y pouvant être identiques ou différents lorsque n est plus grand que 1.

Z représente un radical alkyle inférieur ou alcoxy inférieur (de préférence méthyle, éthyle, méthoxy).

m = 0, 1, 2, les groupements Z étant identiques ou différents lorsque m = 2.

Au sens du présent texte, l'adjectif inférieur, lorsqu' il qualifie un radical organique, signifie que ce radical comporte au plus six atomes de carbone. Ce radical peut être linéaire ou ramifié.

Les composés de formule (I) peuvent former divers sels d'addition avec des acides appropriés pouvant être minéraux, tels que par exemple l'acide chlorhydrique, l'acide sulfurique, l'acide phosphorique, ou organiques tels que par exemple l'acide succinique, l'acide fumarique, l'acide maléique, l'acide oxalique ou l'acide tartrique. Ces divers sels sont compris dans le cadre de la présente invention tout particulièrement lorsqu'ils sont acceptables en agriculture (c'est à dire acceptables pour les plantes traitées).

Ils peuvent être préparés selon des méthodes en soi connues, par exemple en dissolvant le composé (I) dans un solvant approprié, puis en faisant agir un acide approprié.

Les composés selon la formule (I) comportent tous, un atome de carbone substitué de façon asymétrique situé en position alpha par rapport au groupe pyridyle. De ce fait, chacun des composés selon la formule (I) peut exister sous plusieurs formes stéréoisomères, dont les niveaux d'activité antifongique peuvent différer entre eux. Ces formes stéréoisomères ainsi que leurs mélanges, optiquement actifs ou racémiques, sont également inclus dans le cadre de la présente invention.

Parmi les composés de formule (I) décrits précédemment on préfèrera les variantes suivantes prises seules ou en combinaison :

R représente
- un radical alkyle inférieur éventuellement substitué par un ou plusieurs substituants choisis parmi les atomes d' halogène, de préférence le chlore, le fluor, un radical alkoxy inférieur, un radical -Si $(CH_2)_x$ $R_1$ $R_2$ $R_3$, x = 0, 1, $R_1$, $R_2$, $R_3$ identiques ou différents étant alkyle inférieur, alcoxy inférieur, aryle (notamment phényle), aralkyle (notamment benzyle);
- un radical alcényle, de préférence allylique, éventuellement substitué comme lorsque R représente alkyle ou par un groupe alkyle de $C_1$ à $C_4$ ;

A représente un radical alkylène linéaire comportant au plus 4 atomes de carbone, de préférence méthylène ou diméthylène ou propylène ;

X = 0

n = 1, 2, 3

Y = haloalkoxy inférieur, de préférence comportant 1 à 3 atomes de carbone, avantageusement trifluorométhoxy, haloalkyle inférieur, de préférence comportant 1 à 3 atomes de carbone, avantageusement trifluorométhyle, halogène (de préférence chlore ou brome);

m = 0

De plus, il a été trouvé qu'il était particulièrement avantageux dans le cadre des applications précitées d'utiliser les composés de formule (II) dans laquelle :

R, n et Y ont la même signification que dans la formule (I) et de préférence

R représente
- un radical alkyle inférieur éventuellement substitué par un ou plusieurs substituants choisi parmi les atomes d' halogène, de préférence le chlore, le fluor ou le brome, un radical alkoxy inférieur, un radical -Si $(CH_2)_x$ $R_1$, $R_2$, $R_3$, x=0,1, $R_1$, $R_2$, $R_3$ identiques ou différents étant alkyle inférieur, alcoxy inférieur, aryle (notamment phényle), aralkyle (notamment benzyle) ;
- un radical alcényle, de préférence allylique, éventuellement substitué comme lorsque R représente alkyle ou par un groupe alkyle de $C_1$ à $C_3$ ;

Y = haloalkoxy inférieur, de préférence comportant 1 à 3 atomes de carbone, avantageusement trifluorométhoxy, haloalkyle inférieur, de préférence comportant 1 à 3 atomes de carbone, avantageusement trifluorométhyle, halogène (de préférence chlore ou brome).

n = 1, 2, 3, Y pouvant être identique ou différent lorsque n = 2, 3.

Compte tenu des solutions proposées ci-dessus prises séparemment ou en combinaisons on a trouvé qu'il était préférable en raison des propriétés fongicides d'utiliser les composés de formule (I) ou (II) dans lesquelles :

Y est un atome d' halogène, de préférence le brome ou le chlore.

Compte tenu des solutions proposées ci-dessus prises séparemment ou en combinaisons on a trouvé qu'il était préférable en raison des propriétés fongicides d'utiliser les composés de formule (I) ou (II) dans lesquelles n = 1 ou 2.

Compte tenu des solutions proposées ci-dessus prises séparemment ou en combinaisons on a trouvé qu'il était préférable en raison des propriétés fongicides d'utiliser les composés de formule (I) ou (II) dans lesquelles Y est en position 3 et/ou 4.

Compte tenu des solutions proposées ci-dessus prises séparemment ou en combinaisons on a trouvé qu'il était préférable en raison des propriétés fongicides d'utiliser les composés de formule (I) ou (II) dans lesquelles R est un radical $C_1$-$C_6$ alkyle.

L'invention a également pour objet un procédé de préparation des produits selon l'invention.

Dans le cas où A est une chaîne méthylène, un procédé consiste à faire réagir dans une première étape, un composé de formule (III) dans laquelle Z, R, m ont la même signification que dans la formule (I) avec un di-halogénométhylène de formule (IV), Hal représentant un atome d'halogène de préférence le chlore ou le brome, en milieu basique dans un rapport molaire III : IV compris de préférence entre 0,8 et 1,2, éventuellement en présence d'un solvant inerte ce qui conduit au composé de formule (V).

On peut mentionner à titre indicatif des bases minérales comme par exemple la soude ou la potasse, les carbonates de métaux alcalins ou alcalinoterreux, des bases organiques telles que les alcoolates de métaux alcalins comme l'éthanolate de sodium. On utilisera de préférence de 0,5 à 3 équivalents molaires de base..

Comme solvant on peut citer les solvants aprotiques polaires, tels que par exemple, le dibuthyléther, le diisopropyléther, le THF, le diisoamyléther ou le dihalogénométhylène de formule (IV) lui-même. Si désiré, cette réaction peut être effectuée en présence d'un catalyseur approprié. Comme catalyseur utilisable, on peut mentionner des catalyseurs de transfert de phases, tels que par exemple, des dérivés d'ammonium quaternaires comme le chlorure de tétrabutylammonium ou le chlorure de méthyl trialkyl($C_8$-$C_{10}$)ammonium

La température est de préférence comprise entre 20°C et 100°C ou le reflux du solvant quand il y en a un.

Puis, dans une seconde étape le procédé consiste à faire réagir le composé de formule (V) avec le composé de formule (VI) dans laquelle X, Y, n ont la même signification que dans la formule (I) en présence d'une base forte comme les hydrures de métal alcalin ou alcoolates de métal alcalin, en présence éventuellement d'un solvant aprotique polaire approprié tels que par exemple, le diméthylformamide, le diméthylacétamide, le diméthylsulfoxyde, l'acétonitrile, la N-méthylpyrrolidone indiqué à la première étape dans un rapport molaire (V) = (VI) de préférence compris entre 0,8 et 1,2 et à une température comprise de préférence entre 20°C et 100°C ou le reflux du solvant s'il y en a un.

Les composés de formule (IV), (VI) se préparent de manière connue. Les composés (III) peuvent se préparer selon la méthode suivante :

On fait réagir un composé de formule (IX) dans laquelle m et Z ont la même signification que dans la formule (I) avec un composé de formule RHal, R ayant la même signification que dans la formule (I) et Hal représentant un atome d'halogène dans un rapport molaire de préférence compris entre 0,8 et 1,2 en présence d'une base forte organique ou minérale et éventuellement d'un solvant aprotique polaire approprié tels que par exemple, le diméthylformamide, le diméthylacétamide, le diméthylsulfoxyde, l'acétonitrile, la N-méthylpyrrolidone à une température comprise avantageusement entre 20°C et 100°C ou le reflux du solvant s'il y en a un.

Comme base forte on peut citer les hydrures de métal alcalin, les alcoolates de métal alcalin, la soude ou la potasse.

Si désiré, cette réaction peut être effectuée en présence d'un catalyseur approprié. Comme catalyseur utilisable, on peut mentionner des catalyseurs de transfert de phases, tels que par exemple, des dérivés d'ammonium quaternaire come le chlorure de tétrabutylammonium ou le chlorure de méthyl trialkyl ($C_8$-$C_{10}$) ammonium.

Dans le cas où A est différent de méthylène, un procédé consiste dans une première étape à faire réagir le composé de formule (IX) avec un composé de formule (VII) où A est différent de méthylène et Hal correspond à un atome d'halogène en présence d'une base forte dans un rapport molaire (VII) : (IX) compris de préférence entre 0,8 et 1,2, éventuellement en présence d'un solvant aprotique polaire, ce qui conduit au composé de formule (VIII) ; puis dans une seconde étape à faire réagir le composé de formule (VIII) avec un composé de formule Hal-R, Hal étant un atome d'halogène et R ayant la même signification que dans la formule (I) sauf H, en milieu basique dans un rapport molaire compris de préférence entre 0,8 et 1,2 éventuellement en présence d'un solvant aprotique polaire comme indiqué à l'étape 2 et à une température comprise entre 20°C et 100°C ou le reflux du solvant s'il y en a un.

En fin de réaction, quelque soit le procédé utilisé, le composé formé est isolé du milieu réactionnel par les moyens usuels, tel que, par exemple, distillation du solvant, ou par cristallisation du produit dans le milieu réactionnel, ou par filtration, puis si nécessaire, il est ensuite purifié, par exemple par recristallisation dans un solvant approprié, ou par chromatographie.

L'invention a également pour objet les composés éventuellement utilisables à titre d'intermédiaires dans les procédés de préparation décrit ci-dessus et de formule V, VIII dans laquelle Z, R, Y, X, n, m ont l'une quelconque des significations indiquées dans la description ci-avant.

La présente invention concerne également l'utilisation des composés de formule I à titre de fongicides.

Les composés selon l'invention peuvent être utilisés pour la lutte tant préventive que curative contre les champignons, notamment de type basidiomycètes, ascomycètes, adelomycètes ou fungi-imperfecti, en particulier les rouilles, oïdium, piétin-verse, fusarioses, helminthosporioses, septorioses, rhizoctones des végétaux et des plantes en général et en particulier des céréales telles que le blé, l'orge, le seigle, l'avoine et leurs hybrides et aussi le riz et le maïs. Les composés selon l'invention sont actifs en particulier contre les champignons notamment de type basidiomycètes, ascomycètes, adelomycètes ou fungi-imperfecti comme Botrytis cinerea, Erysiphe graminis, Puccinia recondita, Piricularia oryzae, Cercospora beticola, Puccinia striiformis, Erysiphe cichoracearum, Fusarium oxysporum (melonis), Pyrenophora avenae, Septoria

tritici, Venturia inaequalis, Whetzelinia sclerotiorum, Monilia laxa, Mycosphaerella fijiensis, Marssonina panettoniana, Alternaria solani, Aspergillus niger, Cercospora arachidicola, Cladosporium herbarum, Helminthosporium oryzae, Penicillium expansum, Pestalozzia sp, Phialophora cinerescens, Phoma betae, Phoma foveata, Phoma lingam, Ustilago maydis, Verticillium dahliae, Ascochyta pisi, Guignardia bidwellii, Corticium rolfsii, Phomopsis viticola, Sclerotinia sclerotiorum, Sclerotinia minor, Coryneum cardinale, Rhizoctonia solani, Uncinula necator, Podosphaera leucotricha, Fusicladium sp.

Ils sont aussi et encore actifs contre les champignons suivants : Acrostalagmus koningi, les Alternaria, les Colletotrichum, Corticium rolfsii, Diplodia natalensis, Gaeumannomyces graminis, Gibberella fujikuroi, Hormodendron cladosporioides, Lentinus degener ou tigrinus, Lenzites quercina, Memnoniella echinata, Myrothecium verrucaria, Paecylomyces varioti, Pellicularia sasakii, Phellinus megaloporus, Polystictus sanguineus, Poria vaporaria, Sclerotium rolfsii, Stachybotris atra, les Stereum, Stilbum sp. Trametes trabea, Trichoderma pseudokoningi, Trichothecium roseum.

Les composés de l'invention sont spécialement intéressants par leur spectre large au niveau des maladies des céréales (oïdium, rouille, piétin-verse, helminthosporioses, septorioses et fusarioses). Ils présentent également un grand intérêt en raison de leur activité sur la pourriture grise (Botrytis) et les piricularioses, et, de ce fait, ils peuvent être appliqués sur des cultures aussi variées que la vigne, les cultures maraîchères et l'arboriculture et les cultures tropicales telles que l'arachide, le bananier, le caféier, la noix de pécan, le riz et d'autres.

Vu leur absence de phytotoxicité les composés peuvent être utilisés pour la protection des produits de multiplication des plantes contre les maladies causées par des champignons.

L'invention concerne donc en outre un produit de multiplication des plantes cultivées qui a subi un traitement de protection par un composé de l'invention.

On désigne par le nom "produit de multiplication" toutes les parties génératives de la plante qu'on peut utiliser pour la multiplication de celle-ci. On citera par exemple les graines (semences au sens étroit), les racines, les fruits, les tubercules, les bulbes, les rhizomes, les parties de tige, les plants, (pousses) et autres parties de plantes. On mentionnera également les plantes germées et les jeunes plants qui doivent être transplantés aprés germination ou aprés la sortie de terre. Ces jeunes plants peuvent être protégés avant la transplantation par un traitement total ou partiel par immersion.

Ainsi, ces composés peuvent être utilisés dans le traitement des semences (par exemple céréales, cotonnier, betterave, colza, graines fourragères, graines légumières) par exemple sous la forme d'enrobage ou de pelliculage. On pourra trouver dans US 3,989,501, col 7, 1 17-23, une forme d'application. De même, dans FA-A-2 588 442. On pourra également utiliser des suspensions concentrées.

En général ces formulations sont déja connues voir par exemple "catalogue of pesticide formulation types and international coding system" édité par le GIFAP technical monograph n°2, pages 12 à 14, révisée en janvier 1984.

Outre les applications déjà décrites plus haut, les produits selon l'invention présentent en outre une excellente activité biocide à l'égard de nombreuses autres variétés de microorganismes parmi lesquelles on peut citer à titre non limitatif, des champignons comme ceux des genres :

- Pullularia comme l'espèce P. pullulans,
- Chaetomium comme l'espèce C. globosum,
- Aspergillus comme l'espèce Aspergillus niger,
- Coniophora comme l'espèce C. puteana.

En raison de leur activité biocide, les produits de l'invention permettent de combattre efficacement les microorganismes dont la prolifération crée de nombreux problèmes dans les domaines agricole et industriel. A cet effet, ils conviennent tout spécialement bien à la protection des végétaux ou de produits industriels tels que le bois, le cuir, les peintures, le papier, les cordages, les plastiques, les circuits d'eau industriels.

Ils sont tout particulièrement bien adaptés à la protection des produits lignocellulosiques et notamment du bois, qu'il s'agisse de bois d'ameublement, de charpente ou de bois exposé aux intempéries tels que les bois de clôture, les piquets de vignes, les traverses de chemin de fer.

Les composés selon l'invention utilisés seuls ou sous la forme de compositions telles que définies ci-dessus dans les traitements du bois, sont généralement mis en oeuvre avec des solvants organiques et peuvent être éventuellement associés à un ou plusieurs produits biocides connus tels que le pentachlorophénol, les sels métalliques, notamment de cuivre, de manganèse, de cobalt, de chrome, de zinc dérivés d'acides minéraux ou carboxyliques (acides heptanoïque, octanoïque, naphténique); les complexes organiques de l'étain, le mercaptobenzothiazole, les insecticides tels que les pyrethroïdes ou les organochlorés.

Ils présentent enfin une excellente sélectivité vis-à-vis des cultures.

Ils s'appliquent avantageusement à des doses de 0,005 à 5 kg/ha, et plus spécifiquement de 0,01 à 1 kg/ha.

Pour leur emploi pratique, les composés selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active un composé selon l'invention tel que décrit précédemment en association avec les supports solides ou liquides, acceptables en agriculture et/ou les agents tensio-actifs également acceptables en agriculture. En particulier sont utilisables les supports inertes et usuels et les agents tensio-actifs usuels.

Ces compositions contiennent habituellement entre 0,5 et 95% de composé selon l'invention.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,01 à 20 % de matière active.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Par exemple, en plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

Les doses d'emploi dans le cas d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

A titre d'exemple, voici la composition de quelques concentrés :

Exemple F (formulation) 1

| | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique q.s.p | 1 litre |

Selon une autre formule de concentré émulsionnable, on utilise :

Exemple F 2 :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

Exemple F 3 :

| | |
|---|---|
| - matière active | 50 % |
| - lignosulfonate de calcium (défloculant) | 5 % |
| - isopropylnaphtalène sulfonate (agent mouillant anionique) | 1 % |
| - silice antimottante | 5 % |
| - kaolin (charge) | 39 % |

Une autre composition de poudre à pulvériser à 70 % utilise les constituants suivants :

Exemple F 4 :

| | |
|---|---|
| - matière active | 700 g |
| - dibutylnaphtylsulfonate de sodium | 50 g |
| - produit de condensation en proportions 3/2/1 d'acide naphtalène sulfonique, d'acide phénolsulfonique et de formaldéhyde | 30 g |
| - kaolin | 100 g |
| - craie de champagne | 120 g |

Une autre composition de poudre à pulvériser à 40 % utilise les constituants suivants :

Exemple F 5 :

| | |
|---|---|
| - matière active | 400 g |
| - lignosulfonate de sodium | 50 g |
| - dibutylnaphtalène sulfonate de sodium | 10 g |
| - silice | 540 g |

Une autre composition de poudre à pulvériser à 25 % utilise les constituants suivants :

Exemple F 6 :

| | |
|---|---|
| - matière active | 250 g |
| - lignosulfonate de calcium | 45 g |
| - mélange équipondéral de craie de Champagne et d'hydroxyéthylcellulose | 19 g |
| - dibutylnaphtalène sulfonate de sodium | 15 g |
| - silice | 195 g |
| - craie de Champagne | 195 g |
| - kaolin | 281 g |

Une autre composition de poudre à pulvériser à 25 % utilise les constituants suivants :

Exemple F 7 :

| | |
|---|---|
| - matière active | 250 g |
| - isooctylphénoxy-polyoxyéthylène-éthanol | 25 g |
| - mélange équipondéral de craie de Champagne et d'hydroxyéthylcellulose | 17 g |
| - aluminosilicate de sodium | 543 g |
| - kieselguhr | 165 g |

Une autre composition de poudre à pulvériser à 10 % utilise les constituants suivants :

Exemple F 8 :

| | |
|---|---|
| - matière active<br>- mélange de sels de sodium de sulfates d'acides gras saturés<br>- produit de condensation d'acide naphtalène sulfonique et de formaldéhyde<br>- kaolin | 100 g<br>30 g<br>50 g<br>820 g |

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Selon un exemple de composition de granulé, on utilise les constituants suivants :

Exemple F 9 :

| | |
|---|---|
| - matière active<br>- épichlorhydrine<br>- éther de cétyle et de polyglycol<br>- polyéthylène glycol<br>- kaolin (granulométrie : 0,3 à 0,8 mm) | 50 g<br>2,5 g<br>2,5 g<br>35 g<br>910 g. |

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Les exemples ci-après, illustrent la préparation des composés selon l'invention, ainsi que leur utilisation pour la lutte contre les champignons phytopathogènes. Les structures des composés décrits dans ces exemples ont été confirmées par spectrométrie de résonance magnétique nucléaire (R.M.N.) et/ou par spectrométrie infra-rouge.

Exemple 1

Etape 1 : Préparation du 2-(3-pyridinyl) 2-éthyl 3- bromo propanenitrile

On utilise un ballon tricol muni d'un agitateur d'un thermomètre, d'un réfrigérant, d'une ampoule de coulée.

Dans le ballon, on charge successivement 14,6 g de 2-(3-pyridinyl) butane nitrile, 30 ml de bromure de méthylène et 3 g de chlorure de méthyltrialkyl ($C_8$-$C_{10}$)ammonium (Adogen 464), et le mélange est maintenu sous agitation.

On ajoute alors 12 g de soude en solution dans 12 ml d'eau.

Le mélange est porté à 90°C pendant 3 h. Après refroidissement, 20 ml d'eau sont ajoutés à la solution brunâtre qui est extraite au chlorure de méthylène.

Les phases organiques rassemblées sont lavées avec de l'eau, séchées sur sulfate de magnésium puis concentrées.

L'huile brute obtenue est chromatographiée sur colonne de silice (éluant : éther diisopropylique).

On obtient 19,6 g de 2-(3-pyridinyl) 2-éthyl 3-bromo propanenitrile sous la forme d'une huile visqueuse jaune clair. Rendement : 82 %.

Ci-après sont données les caractéristiques des composés intermédiaires de formule XI obtenus de la même manière.

| Rc | Aspect | Rendement |
|---|---|---|
| Et | huile visqueuse | 82 |
| allyl | ,, | 73 |
| i-Pr | ,, | 67 |
| n-Pr | ,, | 61 |
| $CH_2$ Si $Me_3$ | F = 93,9°C | 70 |
| n-Bu | huile visqueuse | 71 |

Etape 2 : Préparation du 2-(3-pyridinyl) 2-éthyl 3-(4-chlorophénoxy) propanenitrile

On utilise le dispositif décrit dans l'exemple précédent. Dans le ballon, on introduit 100 ml de diméthylformamide puis 3,75 g (0,126 at. g) d'hydrure de sodium (à 80 % dans l'huile). Sur la suspension refroidie à 10°C, on coule 15,4 g (0,12 mole) de 4-chlorophénol dilué dans 30 ml de diméthylformamide. Le mélange est maintenu 30 mn à température ambiante.

On ajoute alors 23,9 g (0,1 mole) de 2-(3-pyridinyl)-2 éthyl 3-bromo propanenitrile dilués dans 20 ml de diméthylformamide. La solution est portée à 100°C pendant 6 heures. Après refroidissement à 10°C, on ajoute 300 ml d'eau glacée à la solution, puis on extrait à l'acétate d'éthyle (3 x 60 ml). Les phases organiques rassemblées sont lavées successivement par 100 ml d'une solution aqueuse à 10 % de $KHCO_3$ puis 200 ml d'eau et séchées sur sulfate de magnésium puis concentrées.

Le résidu pâteux obtenu est chromatographié sur colonne de silice (éluant éther diisopropylique). On obtient un solide beige qui est recristallisé dans 40 ml d'un mélange éther diisopropyliquem / heptane 50/50 et conduit à 18,6 g (0,065 mole) de 2-(3-pyridinyl) 2-éthyl 3-(4-chlorophénoxy) propanenitrile sous la forme d'un solide blanc fondant à 82,1°C.

Rendement (calculé à partir du 2-(3-pyridinyl)
2-éthyl3-bromo propanenitrile) : 65 %.

De la même façon ont été obtenus les composés des exemples 2 à 25 qui sont réunis dans le tableaux ci-joint et dont la nomenclature est indiquée aprés les exemples.

TABLEAU I

Composés répondant à la formule générale

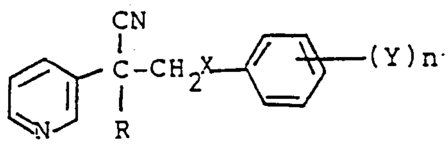

| Composé n° | R | X | 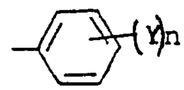 | Point de fusion °C | Rendement % |
|---|---|---|---|---|---|
| 1 | Et | O | p-Cl(Ph) | 82,1 | 65 |
| 2 | Et | S | p-Cl(Ph) | 51,3 | 79 |
| 3 | Et | S | p-F(Ph) | miel | 51 |
| 4 | Et | S | p-Br(Ph) | 58 | 35 |
| 5 | Et | S | m,p-$Cl_2$(Ph) | 79,9 | 52 |
| 6 | Et | O | p-Br(Ph) | 93,6 | 78 |
| 7 | Et | O | m,p-$Cl_2$(Ph) | miel | 69 |
| 8 | Et | O | o,p-$Cl_2$(Ph) | 70,7 | 65 |
| 9 | Et | O | o,o,p-$Cl_3$(Ph) | 37,1 | 48 |
| 10 | allyl | O | p-Cl(Ph) | 57,8 | 78 |
| 11 | allyl | O | o,p-$Cl_2$(Ph) | miel | 62 |
| 12 | allyl | O | p-Br(Ph) | 62,8 | 66 |
| 13 | i-Pr | O | p-Br(Ph) | 62,4 | 70 |
| 14 | allyl | O | m,p-$Cl_2$(Ph) | miel | 65 |
| 15 | i-Pr | O | m,p-$Cl_2$(Ph) | miel | 60 |
| 16 | Et | O | m,m-$Cl_2$(Ph) | 94,2 | 72 |
| 17 | n-Pr | O | p-Cl(Ph) | 77,9 | 61 |
| 18 | n-Pr | O | m,p-$Cl_2$(Ph) | miel | 54 |
| 19 | Et | O | p-$OCF_3$(Ph) | miel | 75 |
| 20 | $CH_2$-Si(Me)3 | O | p-Cl(Ph) | 76,9 | 32 |
| 21 | $CH_2$-Si(Me)3 | O | m,p-$Cl_2$(Ph) | 90,2 | 61 |
| 22 | Et | O | p-F(Ph) | 79,2 | 63 |
| 23 | Et | O | m-F(Ph) | 61,7 | 51 |
| 24 | Et | O | m,m,p-$Cl_3$(Ph) | 116,1 | 50 |
| 25 | Et | O | o,p-$Br_2$(Ph) | 101,2 | 51 |

Ph : noyau phénylène

o : ortho substitué m : méta substitué p : para substitué

Exemple 26

Etape 1 : Préparation du 2-(-3 pyridinyl) 4-(4-chlorophénoxy) butanenitrile

On utilise un ballon tricol muni d'un agitateur et équipé d'un thermomètre, d'un réfrigérant et d'une ampoule de coulée.

Dans le ballon on introduit 250 ml de DMF anhydre puis 3,15 g (0,105 mole) d'hydrure de sodium (à 80 % dans huile).

On ajoute alors goutte à goutte 11,8 g(0,1 mole) de (3-pyridinyl) acétonitrile dilué dans 30 ml de DMF.

Sur le sel formé, on ajoute ensuite goutte à goutte 23,5 g (0,1 mole) de 1-bromo 2-(4-chlorophénoxy) éthane dilué dans 30 ml de DMF.

La solution est maintenue 2 heures sous agitation à 25 °C. Le mélange est alors versé sur 500 ml d'eau glacée puis extrait avec 3 x 150 ml d'acétate d'éthyle. Les phases organiques rassemblées sont lavées avec 300 ml d'eau, séchées sur sulfate de magnésium puis concentrées.

L'huile brute obtenue est reprise par 100 ml d'éther éthylique.

Le précipité blanc qui apparaît rapidement est filtré sur fritté, masse recueillie 3,5 g fondant à 141 °C. Il s'agit du dérivé disubstitué de formule (X)

Le filtrat concentré est chromatographié sur colonne de silice. On obtient 16,8 g de 2-(3-pyridinyl) 4-(4-chlorophénoxy) butanenitrile sous la forme d'une huile jaune clair. Rendement : 62 %.

Etape 2 : Préparation du 2-(3-pyridinyl) 2-méthyl 4-(4-chlorophénoxy) butanenitrile

On utilise le même dispositif que celui décrit dans l'étape 1. Dans le ballon on introduit 100 ml de DMF anhydre puis 1,57 g (5,25 $10^{-2}$ at. g) d'hydrure de sodium.

On ajoute alors goutte à goutte 13,6 g (0,05 mole) de 2-(3-pyridinyl) 4-(4-chlorophénoxy) butanenitrile dilué dans 20 ml de DMF. On coule ensuite 3,2 ml (0,05 mole) d'iodure de méthyle dilué dans 20 ml de DMF.

La solution est maintenue 3 heures sous agitation à 25 °C.

Le mélange est alors versé sur 300 ml d'eau glacée puis extrait avec 3 x 60 ml d'acétate d'éthyle. Les phases organiques rassemblées sont lavées avec 150 ml d'eau, séchées sur sulfate de magnésium puis concentrées.

L'huile brute obtenue est chromatographiée sur colonne de silice (éluant : éther diisopropylique) et conduit à 7,7 g de 2-(3-pyridinyl) 2-méthyl 4-(4-chlorophénoxy) butanenitrile sous la forme d'une huile jaune. Rendement : 54 %.

TABLEAU II

Composés répondant à la formule générale

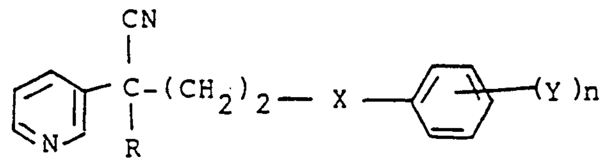

| Composé n° | R | X | 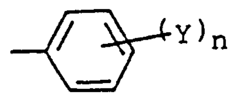 | Aspect | Rendement % |
|---|---|---|---|---|---|
| 26 | H | O | p-Cl(Ph) | huile | 62 |
| 27 | Me | O | p-Cl(Ph) | huile | 54 |
| 28 | Et | O | p-Cl(Ph) | huile | 31 |
| 29 | n-Pr | O | p-Cl(Ph) | cristaux jaune cl F=46,1°C | 26 |
| 30 | allyl | O | p-Cl(Ph) | huile | 41 |
| 31 | Et | O | m,p-$Cl_2$(Ph) | huile | 44 |
| 32 | n-Pr | O | m,p-$Cl_2$(Ph) | huile | 52 |
| 33 | H | S | p-Cl(Ph) | huile | 40 |
| 34 | Me | S | p-Cl(Ph) | huile | 30 |
| 35 | Et | S | p-Cl(Ph) | huile | 66 |
| 36 | n-Pr | S | p-Cl(Ph) | huile | 37 |
| 37 | H | S | p-Br(Ph) | huile | 45 |
| 38 | Me | S | p-Br(Ph) | cristaux blancs F=68,8°C | 54 |
| 39 | Et | S | p-Br(Ph) | huile | 81 |
| 40 | n-Pr | S | p-Br(Ph) | huile | 88 |

Ph : noyau phénylène

Nomenclature

exemple 2 :

2-(3-pyridinyl) 2-éthyl 3-(4-chlorothiophénoxy) propanenitrile.

exemple 3 :

2-(3-pyridinyl) 2-éthyl 3-(4-fluorothiophénoxy) propanenitrile.

exemple 4 :

2-(3-pyridinyl) 2-éthyl 3-(4-bromothiophénoxy) propanenitrile.

exemple 5 :

2-(3-pyridinyl) 2-éthyl 3-(3,4-dichlorothiophénoxy) propanenitrile.

exemple 6 :

2-(3-pyridinyl) 2-éthyl 3-(4-bromophénoxy) propanenitrile.

exemple 7 :

2-(3-pyridinyl) 2-éthyl 3-(3,4-dichlorophénoxy) propanenitrile.

exemple 8 :

2-(3-pyridinyl) 2-éthyl 3-(2,4-dichlorophénoxy) propanenitrile.

exemple 9 :

2-(3-pyridinyl) 2-éthyl 3-(2,4,6-trichlorophénoxy) propanenitrile.

exemple 10 :

2-(3-pyridinyl) 2-allyl 3-(4-chlorophénoxy) propanenitrile.

exemple 11 :

2-(3-pyridinyl) 2-allyl 3-(2,4-dichlorophénoxy) propanenitrile.

exemple 12 :

2-(3-pyridinyl) 2-allyl 3-(4-bromophénoxy) propanenitrile.

exemple 13 :

2-(3-pyridinyl) 2-méthyléthyl 3-(4-bromophénoxy) propanenitrile.

exemple 14 :

2-(3-pyridinyl) 2-allyl 3-(3,4-dichlorophénoxy) propanenitrile.

exemple 15 :

2-(3-pyridinyl) 2-méthyléthyl 3-(3,4-chlorophénoxy) propanenitrile.

exemple 16 :

2-(3-pyridinyl) 2-éthyl 3-(3,5-dichlorophénoxy) propanenitrile.

exemple 17 :

2-(3-pyridinyl) 2-propyl 3-(4-chlorophénoxy) propanenitrile.

exemple 18 :

2-(3-pyridinyl) 2-propyl 3-(3,4-dichlorophénoxy) propanenitrile.

exemple 19 :

2-(3-pyridinyl) 2-éthyl 3-(4-trifluorométhoxyphénoxy) propanenitrile.

exemple 20 :

2-(3-pyridinyl) 2-méthyltriméthylsilyl 3-(4-chlorophénoxy) propanenitrile.

exemple 21 :

2-(3-pyridinyl) 2-méthyltriméthylsilyl 3-(3,4-dichlorophénoxy) propanenitrile.

exemple 22 :

2-(3-pyridinyl) 2-éthyl 3-(4-fluorophénoxy) propanenitrile.

exemple 23 :

2-(3-pyridinyl) 2-éthyl 3-(3-fluorophénoxy) propanenitrile.

exemple 24 :

2-(3-pyridinyl) 2-éthyl 3-(3,4,5-trichlorophénoxy) propanenitrile.

exemple 25 :

2-(3-pyridinyl) 2-éthyl 3-(2,4-dibromophénoxy) propanenitrile.

exemple 26 :

2-(3-pyridinyl) 4-(4-chlorophénoxy) butanenitrile.

exemple 27 :

2-(3-pyridinyl) 2-méthyl 4-(4-chlorophénoxy) butanenitrile.

exemple 28 :

2-(3-pyridinyl) 2-éthyl 4-(4-chlorophénoxy) butanenitrile.

exemple 29 :

2-(3-pyridinyl) 2-propyl 4-(4-chlorophénoxy) butanenitrile.

exemple 30 :

2-(3-pyridinyl) 2-allyl 4-(4-chlorophénoxy) butanenitrile.

exemple 31 :

2-(3-pyridinyl) 2-éthyl 4-(3,4-dichlorophénoxy) butanenitrile.

exemple 32 :

2-(3-pyridinyl) 2-propyl 4-(3,4-dichlorophénoxy) butanenitrile.

exemple 33 :

2-(3-pyridinyl) 4-(4-chlorothiophénoxy) butanenitrile.

exemple 34 :

2-(3-pyridinyl) 2-méthyl 4-(4-chlorothiophénoxy) butanenitrile.

exemple 35

2-(3-pyridinyl) 2-éthyl 4-(4-chlorothiophénoxy) butanenitrile.

exemple 36 :

2-(3-pyridinyl) 2-propyl 4-(4-chlorothiophénoxy) butanenitrile.

exemple 37 :

2-(3-pyridinyl) 4-(4-bromothiophénoxy) butanenitrile.

exemple 38 :

2-(3-pyridinyl) 2-méthyl 4-(4-bromothiophénoxy) butanenitrile.

exemple 39 :

2-(3-pyridinyl) 2-éthyl 4-(4-bromothiophénoxy) butanenitrile.

exemple 40 :

2-(3-pyridinyl) 2-propyl 4-(4-bromothiophénoxy) butanenitrile.

Exemple - Test in vitro sur champignons des semences et champignons du sol

On étudie l'action des composés selon l'invention sur les champignons suivants responsables des maladies des céréales et autres végétaux :

- 11) Pyrenophorae avenae
- 6) Septoria nodorum
- 12) Helminthosporium teres
- 9) Fusarium roseum
- 8) Fusarium nivale
- 7) Fusarium culmorum
- 13) Rhizoctonia cerealis
- 14) Septoria tritici
- 1) Botrytis cinerea sensible au carbendazime et aux imides cycliques
- 2) Botrytis cinerea résistant au carbendazime et aux imides cycliques
- 5) Pseudocercosporella herpotrichoïdes
- 3) Fusarium oxysporum F. sp melonis
- 4) Rhizoctonia solani
- 10) Helminthosporium gramineum

Les chiffres figurant avant les noms seront utilisés pour représenter ces champignons dans le tableau (III).

Pour chaque essai, on opère de la manière suivante : un milieu nutritif constitué de pomme de terre, de glucose et de gelose (milieu PDA) est introduit en surfusion dans une série de boîtes de Pétri (20 ml par

boîte) après stérilisation à l'autoclave à 120 °C.

Au cours du remplissage des boîtes, on injecte, dans le milieu en surfusion, une solution acétonique de la matière active, pour obtenir la concentration finale désirée.

On prend comme témoin des boites de Pétri analogues aux précédentes, dans lesquelles on coule des quantités similaires d'un milieu nutritif ne contenant pas de matière active.

Après 24 ou 48 h chaque boîte est ensemencée par dépôt d'un fragment de mycelium provenant d'une culture précédente du même champignon.

Les boîtes sont conservées pendant 2 à 10 jours (selon le champignon testé) à 22 °C et on compare alors la croissance du champignon dans les boîtes contenant la matière active à tester, à celle du même champignon dans la boîte utilisée comme témoin.

On détermine ainsi pour chaque composé testé le taux d'inhibition du champignon considéré pour une dose de 30 ppm. Les résultats sont indiqués dans le tableau ci-après.

## TABLEAU III

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| 1 | 95 | 95 | 100 | 50 | 100 | 100 | 95 | 95 | 90 | 100 | 100 | 100 | 95 | |
| 2 | 95 | 95 | 95 | 0 | 90 | 90 | 100 | 80 | 80 | 95 | 95 | 95 | 80 | |
| 3 | 95 | 95 | 90 | 0 | 95 | 90 | 95 | 80 | 80 | 90 | 90 | 90 | 80 | |
| 4 | 95 | 95 | 80 | 0 | 95 | 95 | 100 | 95 | 80 | 90 | 90 | 90 | 80 | |
| 5 | 95 | 95 | 80 | 0 | 95 | 95 | 95 | 90 | 50 | 90 | 90 | 90 | 80 | |
| 6 | 95 | 95 | 95 | 50 | 95 | 95 | 100 | 90 | 90 | 95 | 95 | 95 | 80 | |
| 7 | 95 | 95 | 95 | 50 | 100 | 95 | 95 | 90 | 80 | 90 | 90 | 95 | 90 | |
| 8 | 90 | 95 | 95 | 50 | 100 | 95 | 95 | 80 | 80 | 95 | 95 | 95 | 90 | 80 |
| 9 | 80 | 80 | 0 | 0 | 0 | 0 | 90 | 80 | 80 | 80 | 80 | 80 | 80 | 100 |
| 10 | 95 | 95 | 95 | 50 | 100 | 100 | 100 | 100 | 95 | 95 | 95 | 100 | 90 | |
| 11 | 80 | 80 | 80 | 0 | 90 | 80 | 100 | 95 | 90 | 90 | 95 | 100 | 80 | |
| 12 | 90 | 90 | 90 | 80 | 95 | 90 | 95 | 95 | 90 | 95 | 95 | 95 | 95 | 50 |
| 13 | 95 | 95 | 95 | 90 | 100 | 95 | 95 | 95 | 90 | 95 | 100 | 95 | 90 | |
| 14 | 80 | 90 | 80 | 50 | 90 | 90 | 95 | 90 | 90 | 95 | 95 | 95 | 95 | 50 |
| 15 | 80 | 90 | 0 | 80 | 90 | 90 | 100 | 95 | 95 | 90 | 90 | 90 | 90 | |
| 16 | 80 | 80 | 0 | 0 | 0 | 0 | 90 | 90 | 50 | 0 | 90 | 80 | 0 | |
| 17 | 90 | 90 | 80 | 80 | 80 | 80 | 95 | 95 | 90 | 95 | 95 | 100 | 95 | |
| 18 | 90 | 90 | 0 | 0 | 0 | 0 | 95 | 90 | 80 | 90 | 90 | 90 | 90 | |
| 19 | 95 | 95 | 95 | 0 | 100 | 90 | 100 | 80 | 80 | 95 | 90 | 90 | 80 | 100 |
| 20 | 50 | 50 | 0 | 0 | 0 | 0 | 80 | 80 | 80 | 80 | 80 | 80 | 80 | |

TABLEAU III (suite)

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| 21 | 0 | 0 | 0 | 0 | 0 | 0 | 80 | 80 | 80 | 80 | 80 | 80 | 80 | |
| 22 | 100 | 100 | 100 | 0 | 100 | 100 | 100 | 80 | 80 | 90 | 100 | 100 | 80 | 80 |
| 23 | 100 | 100 | 90 | 0 | 90 | 100 | 95 | 80 | 50 | 90 | 95 | 95 | 50 | 100 |
| 24 | 50 | 50 | 50 | 0 | 90 | 90 | 80 | 80 | 50 | 90 | 80 | 80 | 0 | 50 |
| 25 | 50 | 80 | 80 | 80 | 90 | 80 | 95 | 80 | 80 | 100 | 95 | 95 | 80 | |
| 26 | 0 | 0 | 50 | 80 | 80 | 80 | 90 | 80 | 80 | 90 | 90 | 90 | 90 | 100 |
| 27 | 90 | 90 | 90 | 80 | 100 | 100 | 95 | 90 | 80 | 90 | 90 | 90 | 80 | 50 |
| 28 | 95 | 95 | 90 | 80 | 95 | 95 | 80 | 80 | 80 | 95 | 95 | 95 | 95 | |
| 29 | 90 | 90 | 90 | 80 | 100 | 90 | 100 | 95 | 90 | 95 | 95 | 95 | 95 | |
| 30 | 95 | 95 | 90 | 90 | 100 | 100 | 100 | 90 | 90 | 95 | 100 | 100 | 90 | 80 |
| 31 | 90 | 95 | 80 | 80 | 95 | 100 | 90 | 90 | 50 | 95 | 90 | 90 | 80 | |
| 32 | 80 | 80 | 80 | 50 | 90 | 90 | 95 | 90 | 80 | 90 | 95 | 95 | 80 | |
| 33 | 80 | 80 | 80 | 80 | 90 | 50 | 90 | 50 | 50 | 80 | 80 | 80 | 50 | |
| 34 | 80 | 80 | 80 | 80 | 90 | 90 | 90 | 50 | 50 | 90 | 95 | 95 | 80 | 50 |
| 35 | 80 | 80 | 80 | 0 | 90 | 90 | 90 | 50 | 50 | 90 | 90 | 90 | 80 | |
| 36 | 50 | 50 | 50 | 80 | 80 | 80 | 90 | 50 | 50 | 90 | 95 | 95 | 80 | |
| 37 | 80 | 90 | 50 | 80 | 90 | 90 | 90 | 50 | 50 | 80 | 90 | 90 | 80 | 100 |
| 38 | 80 | 90 | 50 | 0 | 95 | 95 | 90 | 50 | 50 | 90 | 90 | 90 | 80 | 100 |
| 39 | 0 | 80 | 80 | 0 | 90 | 90 | 90 | 80 | 80 | 80 | 90 | 95 | 80 | |
| 40 | 0 | 80 | 80 | 0 | 90 | 90 | 90 | 80 | 80 | 80 | 90 | 95 | 80 | |

Exemple : Test in vivo sur Botrytis Cinerea de la tomate.

On prépare par broyage fin une suspension aqueuse de la matière active à tester ayant la composition suivante :

| | |
|---|---|
| - matière active à tester | 40 mg |
| - Tween 80 (agent tensioactif constitué d'un monolaurate de dérivé polyoxyéthylèné du sorbitan | 0,4 ml |
| - eau | 40 ml |

Cette suspension aqueuse est ensuite diluée par de l'eau pour obtenir la concentration désirée.

Des tomates cultivées en serre (variété Marmande) agées de 60 à 75 jours sont traitées par pulvérisation avec des suspensions aqueuses de la composition décrite à concentration de matière active de 1 g/l (1000 ppm). L'essai est répété deux fois avec chaque concentration.

Après 24 heures, les feuilles sont coupées et mises dans 2 boîtes de Piétri (diamètre 11 cm) dont le fond a été préalablement garni d'une disque de papier filtre humide (5 folioles par boîte).

L'inoculum est ensuite apporté à l'aide d'une seringue par dépôt de gouttes (3 gouttes par foliole) d'une suspension de spores. Cette suspension de spores de Botrytis cinerea a été obtenue à partir d'une culture de 15 jours, mise ensuite en suspension dans une solution nutritive (80.000 unités/$cm^3$). Le contrôle est fait environ 4 à 6 jours après la contamination par comparaison avec un témoin non traité. On évalue ainsi le pourcentage de protection par rapport au témoin non traité.

Dans ces conditions, on observe que, à la concentration de 1 000 mg/l, le pourcentage de protection était respectivement d'au moins 75 % pour les composés suivants : 1, 4, 5, 6, 7, 10, 12, 13, 15, 17, 18, 22, 23, 27, 28, 29, 30, 35.

Test in vivo sur l'oïdium de l'orge en traitement préventif.

On prépare par broyage fin une suspension aqueuse de la matière active à tester ayant la composition suivante :

| | |
|---|---|
| - matière active à tester | 60 mg |
| - Tween 80 (agent tensioactif constitué d'une monolaurate de dérivé polyoxyéthyléné du sorbitan) dilué au 1/10 | 0,3 ml |
| - eau | 60 ml |

Cette suspension aqueuse est ensuite diluée par de l'eau pour obtenir la concentration désirée.

Des plants d'orge (Hordeum vulgare), de variété Berac, sont cultivés en godets. Cinq à six jours après le semis, leur feuillage est traité en pulvérisant sur chacun d'eux 8 ml d'une suspension de la matière active dans de l'eau distillée contenant du Tween 80, à la concentration désirée.

Chaque concentration de la matière active fait l'objet de trois répétitions. Les plants témoins sont traités de la même façon mais sans matière active.

Après ressuyage, le lendemain du traitement, les plantes sont contaminées par saupoudrage au moyen de spores d'oïdium de l'orge (Erysiphe graminis) puis maintenues en serre à 22°C plus ou moins 2°C sous 60 à 80 % d'humidité relative.

Quatorze jours après le traitement par la suspension de la matière active, on évalue le pourcentage d'inhibition du développement du champignon, par comparaison avec le témoin non traité.

Dans ces conditions, on a observé que, à la concentration de 1 000 mg/l, le pourcentage d'inhibition du développement du champignon était respectivement : - d'au moins 75 % pour les composés : 5, 7, 9, 18, 20, 22, 23.

Exemple - Test in vivo sur "Puccina recondita" responsable de la rouille du blé

Du blé, en godets, semé dans de la terre franche, est traité au stade 10 cm de hauteur par pulvérisation avec des émulsions aqueuses (appelées bouillies) de même composition que celle décrite à l'exemple précédent et à diverses concentrations du composé à tester. L'essai est répeté deux fois avec chaque concentration.

Au bout de 24 heures, une suspension aqueuse de spores (50000 sp/$cm^3$) est pulvérisée sur le blé ; cette suspension a été obtenue à partir de plants contaminés. On place ensuite le blé pendant 48 heures en cellule d'incubation à environ 18°C et à 100 % d'humidité relative.

Au bout de ces 2 jours, l'humidité relative est ramenée à 60 %. Le contrôle de l'état des plants se fait entre le 11ème et le 15ème jour après la contamination par comparaison avec le témoin non traité.

A la dose de 1 g/l, protection d'au moins 75 % avec les composés 1, 4, 6, 7, 10, 13, 19, 20, 28, 29.

Exemple - Test in vivo sur "piricularia oryzae" responsable de la Piriculariose du riz (Rice Blast)

Du riz, en godets, semé dans un mélange 50/50 de tourbe enrichie et de pouzzolane, est traité au stade 10 cm de hauteur par pulvérisation d'une émulsion aqueuse (appelée bouillie) ci-dessus définie de concentration indiquée ci-après. L'essai est répété deux fois. Au bout de 24 heures, on traite par application sur les feuilles, par une suspension de spores obtenues en culture pure.

Les plantes ainsi traitées et contaminées sont alors placées en incubation à 25-28°C en humidité saturante pendant 24 heures, puis en chambre humide en serre a 25-28°C.

La lecture se fait 8 jours après la contamination. Dans ces conditions, on observe les résultats suivants : à la dose de 1 g/l, protection d'au moins 75 % avec les composés 1, 2, 4, 6, 7, 8, 9, 10, 11, 12, 13, 14, 15,

16, 20, 22, 23, 24, 29, 30, 32 37, 38.

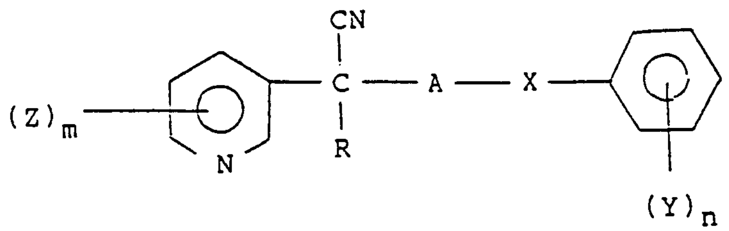

(I)

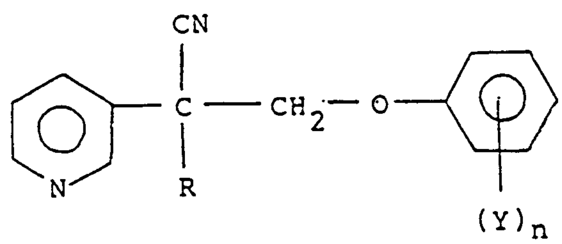

(II)

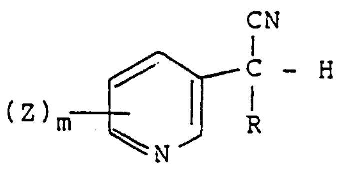

(III)

$(Hal)_2 CH_2$

(IV)

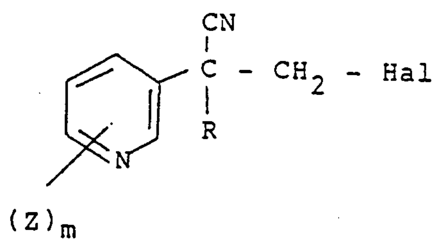

(V)

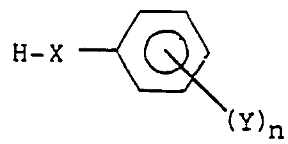

(VI)

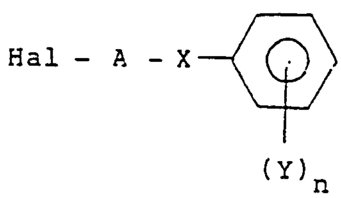

(VII)

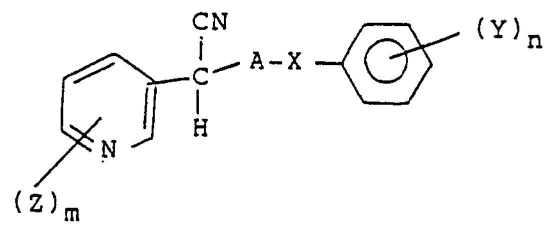

(VIII)

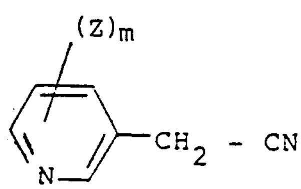

(IX)

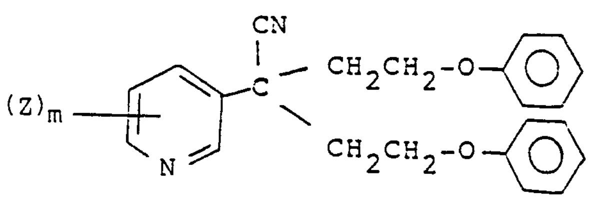

(X)

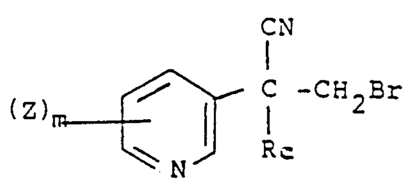

(XI)

## Revendications

**Revendications pour les Etats contractants suivants : BE, CH, DE, FR, GB, IT, LI, LU, NL, SE**

**1.** Composés de formule I:

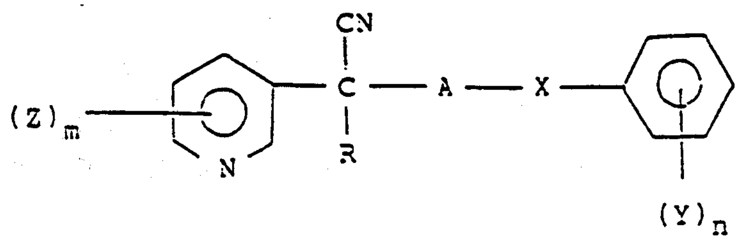

(I)

dans laquelle

- A représente une chaîne alkylène linéaire comportant au plus six atomes de carbone ou ramifiée comportant au plus six atomes de carbone à condition que la chaine linéaire soit différente de méthylène
- R représente :
  - un radical alkyle ayant au plus six atomes de carbone éventuellement substitué par un ou plusieurs substituants choisis parmi les atomes d'halogènes et les radicaux alcoxy ayant au plus six atomes de carbone, alkylthio ayant au plus six atomes de carbone, -Si $(CH_2)_x$ $R_1$ $R_2$ $R_3$, x = 0, 1, $R_1$, $R_2$, $R_3$ identiques ou différents étant alkyle ayant au plus six atomes de carbone, alcoxy ayant au plus six atomes de carbone, aryle, aralkyle ;
  - un radical cycloalkyle comportant 3 à 7 chaînons lui-même éventuellement substitué de la même manière que le radical alkyle ;
  - un radical aralkyle éventuellement substitué de la même manière que le radical alkyle ;
  - un radical alcynyle ou alcényle ayant au plus six atomes de carbone mono ou poly-insaturé éventuellement substitué de la même manière que le groupement alkyle à condition qu'il n'y ait pas d'insaturation en alpha du carbone portant le groupe CN ;

  en outre, R peut être H si A est différent de méthylène.
- X = O ou S
- Y représente un atome d'halogène un radical alkyle ayant au plus six atomes de carbone, un radical haloalkyle ayant au plus six atomes de carbone un radical alcoxy ayant au plus six atomes de carbone, un radical haloalkoxy ayant au plus six atomes de carbone, un radical alkylthio ayant au plus six atomes de carbone, un radical haloalkylthio ayant au plus six atomes de carbone, un radical alkoxy carbonyle ayant au plus six atomes de carbone, un radical haloalkoxy carbonyle ayant au plus six atomes de carbone ayant au plus six atomes de carbone, un radical alcanoyloxy ayant au plus six atomes de carbone, un radical haloalcanoyloxy ayant au plus six atomes de carbone, un radical aryle, un radical aralkyle, un radical cyclohexyle de 3 à 7 atomes de carbone, un radical cyano ou nitro ou hydroxyle.
- n nombre entier positif ou nul, inférieur à 6 les groupements Y pouvant être identiques ou différents lorsque n est plus grand que 1.
- Z représente un radical alkyle ayant au plus six atomes de carbone ou alcoxy ayant au plus six atomes de carbone.
- m = 0, 1, 2, les groupements Z étant identiques ou différents lorsque m = 2.

  et les sels acceptables en agriculture de ces composés

**2.** Composés de formule (I), selon la revendication 1, caractérisés en ce que R représente un radical alkyle ayant au plus six atomes de carbone éventuellement substitué par un ou plusieurs substituants choisis parti les atomes d'halogène, un radical alkoxy ayant au plus six atomes de carbone, un radical -Si $(CH_2)_x$ $R_1$ $R_2$ $R_3$, x = 0, 1,
$R_1$, $R_2$, $R_3$ identiques ou différents étant alkyle ayant au plus six atomes de carbone, alcoxy ayant au plus six atomes de carbone, aryle, aralkyle

- un radical alcényle, éventuellement substitué comme lorsque R représente alkyle ou par un groupe alkyle de $C_1$ à $C_4$ ;

A représente un radical alkylène linéaire comportant au plus 4 atomes de carbone,

X = 0

n = 1, 2, 3
Y = haloalkoxy comportant 1 à 3 atomes de carbone, haloalkyle comportant 1 à 3 atomes de carbone, halogène m = 0

**3.** Composés de formule (II),

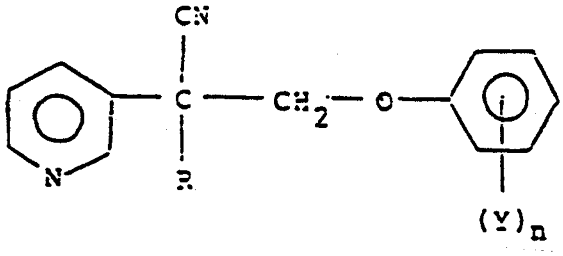

(II)

,

caractérisés en ce que R, n, Y ont la même signification que dans la revendication 1 et, de préférence, R représente un radical alkyle ayant au plus six atomes de carbone éventuellement substitué par un ou plusieurs substituants choisi parmi les atomes d'halogène, un radical alkoxy ayant au plus six atomes de carbone, un radical -Si $(CH_2)_x$ $R_1$ $R_2$ $R_3$, x =0, 1, $R_1$, $R_2$, $R_3$ identiques ou différents étant alkyle ayant au plus six atomes de carbone, alcoxy ayant au plus six atomes de carbone, aryle, aralkyle, un radical alcényle, éventuellement substitué comme lorsque R représente alkyle ou par un groupe alkyle de $C_1$ à $C_3$, Y est haloalkoxy comportant 1 à 3 atomes de carbone, haloalkyle comportant 1 à 3 atomes de carbone, halogène, n = 1, 2, 3, Y pouvant être identique ou différent lorsque n = 2, 3.
et les sels acceptables en agriculture de ces composés

**4.** Composés selon la revendication 1, caractérisés en ce que n = 1 ou 2 et en ce que Y est un atome d'halogène.

**5.** Composés selon la revendication 1, caractérisés en ce que n = 1 ou 2.

**6.** Composés selon la revendication 1, caractérisés en ce que Y est en position 3 et/ou 4.

**7.** Composés selon la revendication 1, caractérisés en ce que R est un radical C1-C6 alkyle.

**8.** Utilisation des composés selon l'une des revendications 1 à 7, à titre de fongicide notamment sous forme de composition fongicide comprenant 0,5 % à 95 % de composés en combinaison avec les supports solides ou liquides acceptables en agriculture et/ou ces agents tensio-actifs également acceptables en agriculture.

**9.** Procédé de traitement des cultures atteintes ou susceptibles d'être atteintes par les maladies fongiques caractérisé en ce que l'on applique sur les feuilles une quantité efficace d'un composé selon l'une des revendications 1 à 7.

**10.** Procédé de traitement selon la revendication 9 caractérisé en ce que l'on applique une dose comprise entre 0,005 et 5 kg/ha

**11.** Composés utiles comme intermédiaires dans la préparation des composés selon les revendications 1 à 7, répondant aux formules( V, VIII)

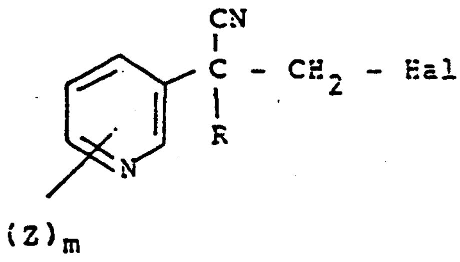

(V)

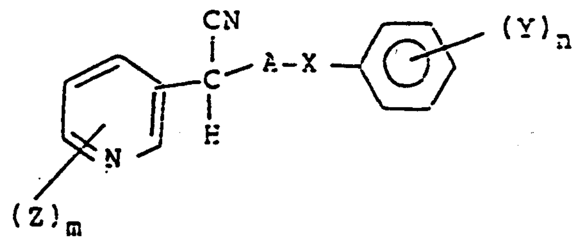

(VIII)

**12.** Procédé de préparation des composés de formule (I) dans le cas où A est méthylène caractérisé en ce que on fait réagir dans une première étape, un composé de formule (III) dans laquelle Z, R, m ont la même signification que dans la avec revendication 1 un di-halogénométhylène de formule (IV), Hal représentant un atome d'halogène, en milieu basique dans un rapport molaire III : IV compris entre 0,8 et 1,2, éventuellement en présence d'un solvant inerte ce que conduit au composé de formule (V).
Puis, dans une seconde étape, le procédé consiste à faire réagir le composé de formule (V) avec le composé de formule (VI) dans laquelle X, Y, n ont la même signification que dans la formule (I) en présence d'une base forte comme les hydrures de métal alcalin ou alcoolates de métal alcalin, en présence éventuellement d'un solvant aprotique polaire approprié.

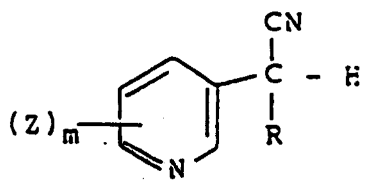

(III)

$(Hal)_2 CH_2$

(IV)

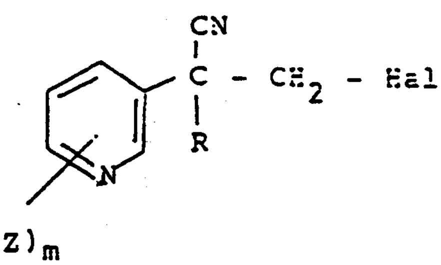

(V)

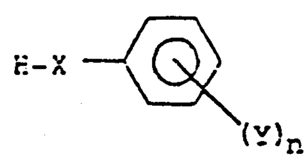

(VI)

**13.** Procédé de préparation des composés de formule (I) dans le cas où A est différent de méthylène caractérisé en ce que le procédé consiste dans une première étape à faire réagir le composé de formule (IX) avec un composé de formule (VII) où A est différent de méthylène et Hal correspond à un atome d'halogène en présence d'une base forte dans un rapport molaire (VII) : (IX) compris entre 0,8 et 1,2, éventuellement en présence d'un solvant aprotique polaire, ce qui conduit au composé de formule (VIII) ; puis dans une seconde étape à faire réagir le composé de formule (VIII) avec un composé de formule Hal-R, Hal étant un atome d'halogène et R ayant la même signification que dans la revendication 1 sauf H

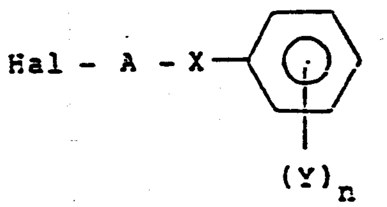

(VII)

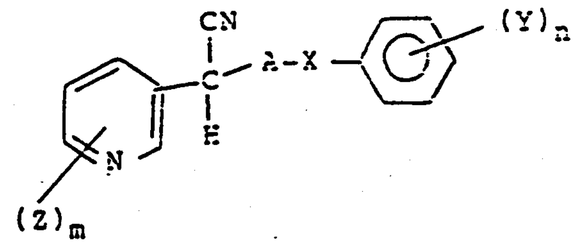

(VIII)

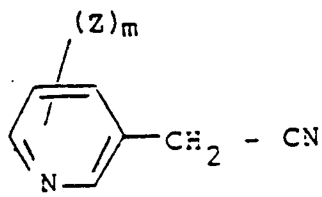

(IX)

**14.** Produit de multiplication des plantes cultivées qui a subi un traitement de protection par un des composés selon l'une des revendications 1 à 7.

**15.** Semence ayant subi un traitement de protection par un des composés selon l'une de revendications 1 à 7.

**Revendications pour les Etats contractants suivants : AT, ES, GR**

**1.** Composition fongicide contenant un ou des composés de formule (I) dans laquelle :

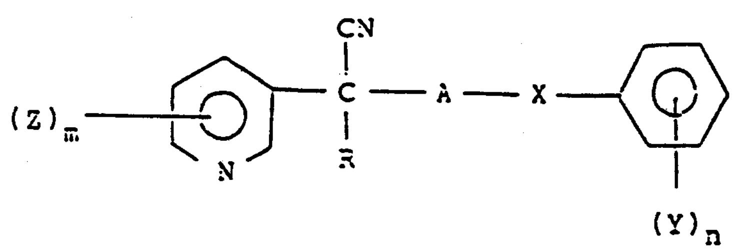

(I)

A représente une chaîne alkylène linéaire comportant au plus six atomes de carbone ou ramifiée comportant au plus six atomes de carbone à condition que la chaine linéaire soit différente de méthylène,

R représente :

- un radical alkyle ayant au plus six atomes de carbone éventuellement substitué par un au plusieurs substituants choisis parmi les atomes d'halogènes et les radicaux alcoxy ayant au plus six atomes de carbone, alkylthio ayant au plus six atomes de carbone, -Si $(CH_2)_x$ $R_1$ $R_2$ $R_3$, x = 0, 1, $R_1$, $R_2$, $R_3$ identiques ou différents étant alkyle ayant au plus six atomes de carbone, alcoxy ayant au plus six atomes de carbone, aryle, aralkyle ;
- un radical cycloalkyle comportant 3 à 7 chaînons lui-même éventuellement substitué de la même manière que le radical alkyle ;
- un radical aralkyle éventuellement substitué de la même manière que le radical alkyle ;

- un radical alcynyle ou alcényle ayant au plus six atomes de carbone mono ou poly-insaturé éventuellement substitué de la même manière que le groupement alkyle à condition qu'il n'y ait pis d'insaturation en alpha du carbone portant le groupe CN ;

en outre, R peut être H si A est différent de méthylène.

X = O ou S

Y représente un atome d'halogène un radical alkyle ayant au plus six atomes de carbone, un radical haloalkyle ayant au plus six atomes de carbone un radical alcoxy ayant au plus six atomes de carbone, un radical haloalkoxy ayant au plus six atomes de carbone, un radical alkylthio ayant au plus six atomes de carbone, un radical haloalkylthio ayant au plus six atomes de carbone, un radical alkoxy carbonyle ayant au plus six atomes de carbone, un radical haloalkoxy carbonyle ayant au plus six atomes de carbone, un radical alcanoyloxy ayant au plus six atomes de carbone, un radical haloalcanoyloxy ayant au plus six atomes de carbone, un radical aryle un radical aralkyle, un radical cyclohexyle de 3 à 7 atomes de carbone, un radical cyano ou nitro ou hydroxyle.

n nombre entier positif ou nul, inférieur à 6 les groupements Y pouvant être identiques ou différents lorsque n est plus grand que 1.

Z représente un radical alkyle ayant au plus six atomes de carbone ou alcoxy ayant au plus six atomes de carbone.

m = 0, 1, 2, les groupements Z étant identiques ou différents lorsque m = 2.

et les sels acceptables en agriculture de ces composés

**2.** Composition fongicide selon la revendication 1, caractérisés en ce que R représente un radical alkyle ayant au plus six atomes de carbone éventuellement substitué par un ou plusieurs substituants choisis parmi les atomes d'halogène, un radical alkoxy ayant au plus six atomes de carbone, un radical -Si $(CH_2)_x$ $R_1$ $R_2$ $R_3$, x = 0, 1,

$R_1$, $R_2$, $R_3$ identiques ou différents étant alkyle ayant au plus six atomes de carbone, alcoxy ayant au plus six atomes de carbone, aryle, aralkyle ;

- un radical alcényle, éventuellement substitué comme lorsque R représente alkyle ou par un groupe alkyle de $C_1$ à $C_4$ ;

A représente un radical alkylène linéaire comportant au plus 4 atomes de carbone ;

X = 0

n = 1, 2, 3

Y = haloalkoxy comportant 1 à 3 atomes de carbone, haloalkyle comportant 1 à 3 atomes de carbone, halogène

m = 0

**3.** Composition fongicide contenant un ou plusieurs composés de formule (II),

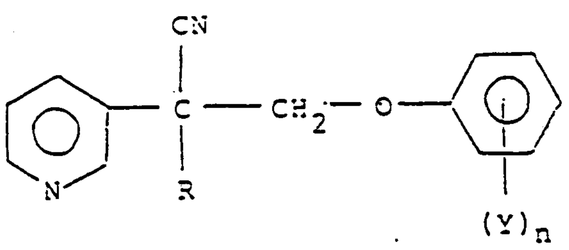

(II)

caractérisés en ce que R, n, Y ont la même signification que dans la revendication 1 et, de préférence, R représente un radical alkyle ayant au plus six atomes de carbone éventuellement substitué par un ou plusieurs substituants choisi parmi les atomes d'halogène, un radical alkoxy ayant au plus six atomes de carbone, un radical -Si $(CH_2)_x$ $R_1$ $R_2$ $R_3$, x =0, 1, $R_1$, $R_2$, $R_3$ identiques ou différents étant alkyle ayant au plus six atomes de carbone, alcoxy ayant au plus six atomes de carbone, aryle, aralkyle, un radical alcényle, éventuellement substitué comme lorsque R représente alkyle ou par un groupe alkyle de $C_1$ à $C_3$, Y est haloalkoxy comportant 1 à 3 atomes de carbone, haloalkyle comportant 1 à 3 atomes de carbone, halogène, n = 1, 2, 3, Y pouvant être identique ou différent lorsque n = 2, 3.

et les sels acceptables en agriculture de ces composés

**4.** Composition fongicide selon la revendication 1, caractérisés en ce que n = 1 ou 2 et en ce que Y est un atome d'halogène.

**5.** Composition fongicide selon la revendication 1, caractérisés en ce que n = 1 ou 2.

**6.** Composition fongicide selon la revendication 1, caractérisés en ce que Y est en position 3 et/ou 4.

**7.** Composition fongicide selon la revendication 1, caractérisés en ce que R est un radical C1-C6 alkyle.

**8.** Procédé de traitement des cultures atteintes ou susceptibles d'être atteintes par les maladies fongiques caractérisé en ce que l'on applique sur les feuilles une quantité efficace d'une composition selon l'une des revendications 1 à 7.

**9.** Procédé de traitement selon la revendication 8 caractérisé en ce que l'on applique une dose de composé de formule (I) comprise entre 0,005 et 5 kg/ha

**10.** Procédé de préparation des composés de formule (I) dans le cas où A est méthylène caractérisé en ce que on fait réagir dans une première étape, un composé de formule (III) dans laquelle Z, R, m ont la même signification que dans la revendication 1 avec un di-halogénométhylène de formule (IV), Hal représentant un atome d'halogène en milieu basique dans un rapport molaire III : IV compris entre 0,8 et 1,2, éventuellement en présence d'un solvant inerte ce qui conduit au composé de formule (V).
Puis, dans une seconde étape, le procédé consiste à faire réagir le composé de formule (V) avec le composé de formule (VI) dans laquelle X, Y, n ont la même signification que dans la formule (I) en présence d'une base forte comme les hydrures de métal alcalin ou alcoolates de métal alcalin, en présence éventuellement d'un solvant aprotique polaire approprié.

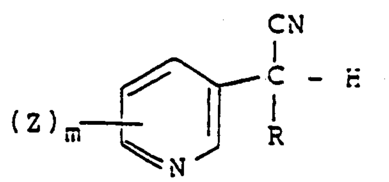

(III)

$(Hal)_2\ CH_2$

(IV)

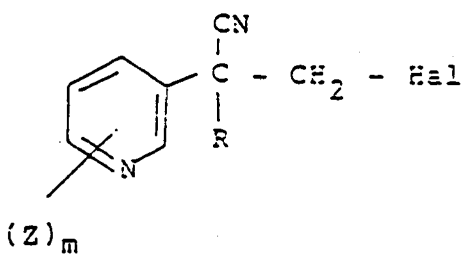

(V)

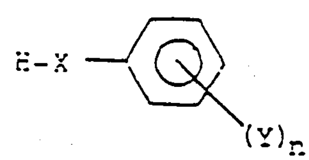

(VI)

**11.** Procédé de préparation des composés de formule (I) dans le cas où A est différent de méthylène caractérisé en ce que le procédé consiste dans une première étape à faire réagir le composé de formule (IX) avec un composé de formule (VII) où A est différent de méthylène et Hal correspond à un atome d'halogène en présence d'une base forte dans un rapport molaire (VII) : (IX) compris entre 0,8 et 1,2, éventuellement en présence d'un solvant aprotique polaire, ce que conduit au composé de formule (VIII) ; puis dans une seconde étape à faire réagir le composé de formule (VIII) avec un composé de formule Hal-R, Hal étant un atome d'halogène et R ayant la même signification que dans la revendication 1 sauf H

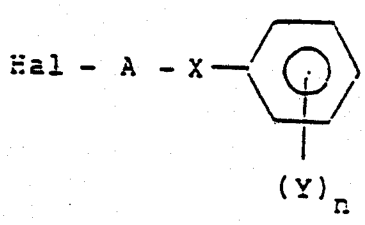

(VII)

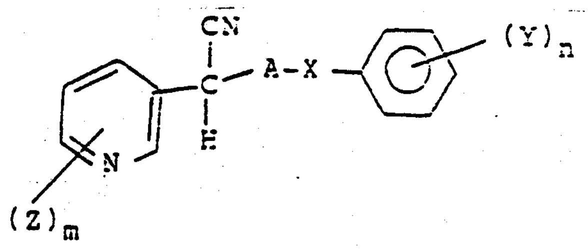

(VIII)

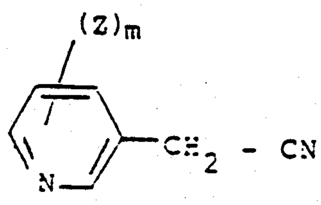

(IX)

**Claims**
**Claims for the following Contracting States : BE, CH, DE, FR, GB, IT, LI, LU, NL, SE**

**1.** Compounds of formula (I):

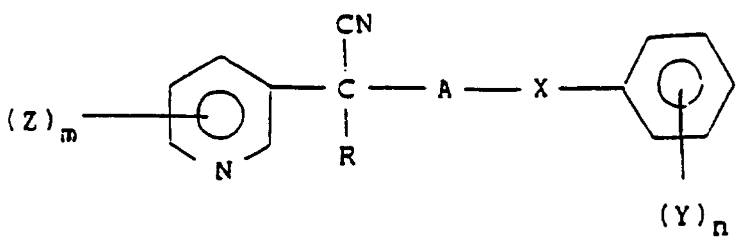

(I)

in which:

A denotes a linear alkylene chain containing at most six carbon atoms, or a branched alkylene chain containing at most six carbon atoms, with the proviso that the linear chain is other than methylene,

R denotes:

- an alkyl radical having at most six carbon atoms, optionally substituted with one or more substituents chosen from halogen atoms, alkoxy and alkylthio radicals, each radical having at most six carbon atoms, and a radical $-Si(CH_2)_xR_1R_2R_3$, x = 0 or 1 and $R_1$, $R_2$ and $R_3$, which may be identical or different, being alkyl having at most six carbon atoms, alkoxy having at most six carbon atoms, aryl or aralkyl;
- a 3- to 7-membered cycloalkyl radical, which is itself optionally substituted in the same manner as the alkyl radical;
- an aralkyl radical, optionally substituted in the same manner as the alkyl radical;
- a mono- or polyunsaturated alkenyl or alkynyl radical having at most six carbon atoms, optionally substituted in the same manner as the alkyl group, with the proviso that there is no unsaturation at the alpha-position with respect to the carbon bearing the CN group; in addition, R may be H if A is other than methylene;

X = O or S;

Y denotes a halogen atom, an alkyl radical having at most six carbon atoms, a haloalkyl radical having at most six carbon atoms, an alkoxy-radical having at most six carbon atoms, a haloalkoxy radical having at most six carbon atoms, an alkylthio radical having at most six carbon atoms, a haloalkylthio radical having at most six carbon atoms, an alkoxycarbonyl radical having at most six carbon atoms, a haloalkoxycarbonyl radical having at most six carbon atoms, an alkanoyloxy radical having at most six carbon atoms, a haloalkanoyloxy radical having at most six carbon atoms, an aryl radical, an aralkyl radical, a cyclohexyl radical containing 3 to 7 carbon atoms, or a cyano or nitro or hydroxyl radical;

n = positive integer less than 6, or zero, it being possible for the groups Y to be identical or different when n is larger than 1;

Z denotes an alkyl or alkoxy radical having at most six carbon atoms;

m = 0, 1 or 2, the groups Z being identical or different when m = 2;

and the salts of these compounds which are acceptable in agriculture.

**2.** Compounds of formula (I) according to claim 1, characterized in that R denotes an alkyl radical having at most six carbon atoms, optionally substituted with one or more substituents chosen from halogen atoms, an alkoxy radical having at most six carbon atoms and a radical $-Si(CH_2)_xR_1R_2R_3$, x = 0 or 1 and $R_1$, $R_2$ and $R_3$, which may be identical or different, being alkyl having at most six carbon atoms, alkoxy having at most six carbon atoms, aryl or aralkyl;

- an alkenyl radical, optionally substituted as when R denotes alkyl or with a $C_1$ to $C_4$ alkyl group;

A denotes a linear alkylene radical containing at most 4 carbon atoms;

X = 0;

n = 1, 2 or 3;

Y = haloalkoxy containing 1 to 3 carbon atoms, haloalkyl containing 1 to 3 carbon atoms, or halogen;

m = 0.

**3.** Compounds of formula (II),

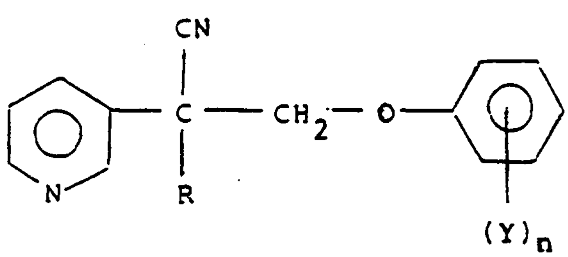

(II),

characterized in that R, n and Y have the same meaning as in claim 1 and, preferably, R denotes an alkyl radical having at most six carbon atoms, optionally substituted with one or more substituents chosen from halogen atoms, an alkoxy radical having at most six carbon atoms and a radical $-Si(CH_2)_xR_1R_2R_3$, x = 0 or 1 and $R_1$, $R_2$ and $R_3$, which may be identical or different, being alkyl having at most six carbon atoms, alkoxy having at most six carbon atoms, aryl or aralkyl, or an alkenyl radical, optionally substituted as when R denotes alkyl or with a $C_1$ to $C_3$ alkyl group, Y is haloalkoxy containing 1 to 3 carbon atoms, haloalkyl containing 1 to 3 carbon atoms, or halogen, n = 1, 2 or 3, it being possible for Y to be identical or different when n = 2 or 3,

and the salts of these compounds which are acceptable in agriculture.

**4.** Compounds according to claim 1, characterized in that n = 1 or 2 and in that Y is a halogen atom.

**5.** Compounds according to claim 1, characterized in that n = 1 or 2.

**6.** Compounds according to claim 1, characterized in that Y is at the 3- and/or 4-position(s).

**7.** Compounds according to claim 1, characterized in that R is a $C_1$-$C_6$ alkyl radical.

**8.** Use of the compounds according to one of claims 1 to 7, by way of a fungicide, in particular in the form of a fungicidal composition comprising 0.5% to 95% of compounds in combination with solid or liquid vehicles which are acceptable in agriculture and/or surfactant agents which are also acceptable in agriculture.

**9.** Process for treating crops attacked, or likely to be attacked, by fungal diseases, characterized in that an effective amount of a compound according to one of claims 1 to 7 is applied on the leaves.

**10.** Treatment process according to claim 9, characterized in that a dose of between 0.005 and 5 kg/ha is applied.

**11.** Compounds which are useful as intermediates in the preparation of the compounds according to claims 1 to 7, the compounds being of the formula (V) or (VIII).

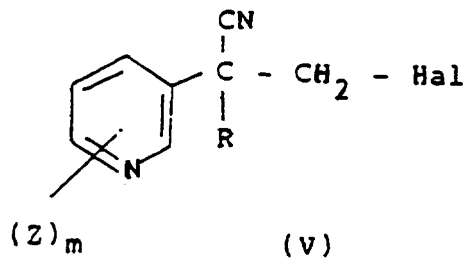

(V)

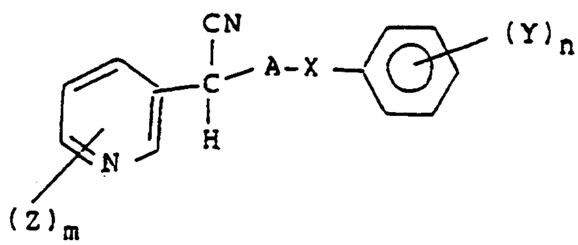

(VIII)

**12.** Process for preparing the compounds of formula (I) in the case where A is methylene, characterized in that, in a first stage, a compound of formula (III) in which Z, R and m have the same meaning as in claim 1 is reacted with a dihalomethylene of formula (IV), Hal denoting a halogen atom, in a basic medium in a mole ratio III/IV of between 0.8 and 1.2, optionally in the presence of an inert solvent, leading to the compound of formula (V).
Then, in a second stage, the process consists in reacting the compound of formula (V) with the compound of formula (VI) in which X, Y and n have the same meaning as in formula (I), in the presence of a strong base such as alkali metal hydrides or alkali metal alcoholates, optionally in the presence of a suitable polar aprotic solvent.

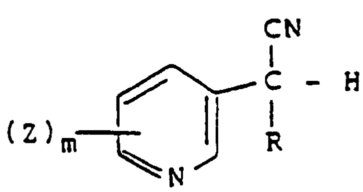

(III)

$(Hal)_2 \; CH_2$

(IV)

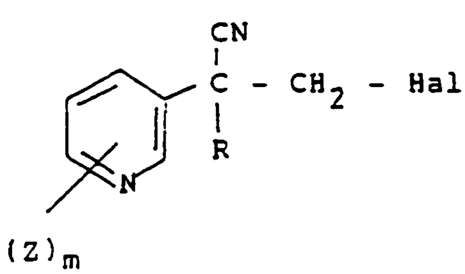

(V)

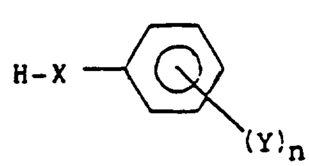

(VI)

**13.** Process for preparing the compounds of formula (I) in the case where A is other than methylene, characterized in that the process consists, in a first stage, in reacting the compound of formula (IX) with a compound of formula (VII) where A is other than methylene and Hal corresponds to a halogen atom, in the presence of a strong base, in a mole ratio (VII)/(IX) of between 0.8 and 1.2, optionally in the presence of a polar aprotic solvent, leading to the compound of formula (VIII); then, in a second stage, in reacting the compound of formula (VIII) with a compound of formula Hal-R, Hal being a halogen atom and R having the same meaning as in claim 1 except for H.

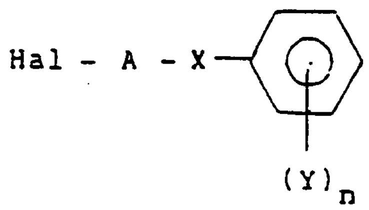

(VII)

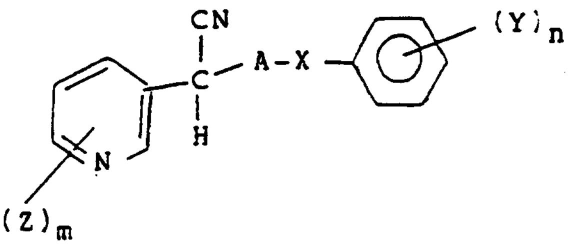

(VIII)

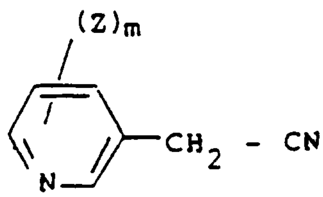

(IX)

**14.** Product of multiplication of cultivated plants, which has undergone a protective treatment with one of the compounds according to one of claims 1 to 7.

**15.** Seed having undergone a protective treatment with one of the compounds according to one of claims 1 to 7.

**Claims for the following Contracting States : AT, ES, GR**

**1.** Fungicidal composition containing one or more compounds of formula (I),

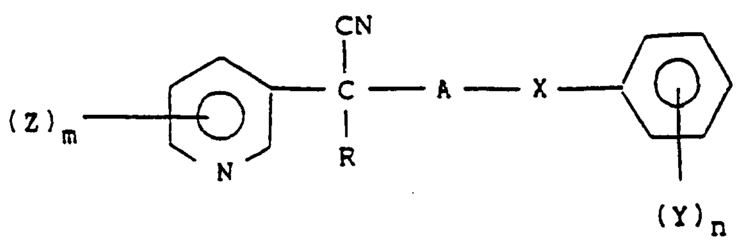

(I)

in which:

A denotes a linear alkylene chain containing at most six carbon atoms, or a branched alkylene chain containing at most six carbon atoms, with the proviso that the linear chain is other than methylene,

R denotes:

- an alkyl radical having at most six carbon atoms, optionally substituted with one or more substituents chosen from halogen atoms, alkoxy and alkylthio radicals, each radical having at most six carbon atoms, and a radical $-Si(CH_2)_xR_1R_2R_3$, x = 0 or 1 and $R_1$, $R_2$ and $R_3$, which may be identical or different, being alkyl having at most six carbon atoms, alkoxy having at most six carbon atoms, aryl or aralkyl;

- a 3- to 7-membered cycloalkyl radical, which is itself optionally substituted in the same manner as the alkyl radical;
- an aralkyl radical, optionally substituted in the same manner as the alkyl radical;
- a mono- or polyunsaturated alkenyl or alkynyl radical having at most six carbon atoms, optionally substituted in the same manner as the alkyl group, with the proviso that there is no unsaturation at the alpha-position with respect to the carbon bearing the CN group; in addition, R may be H if A is other than methylene;

X = O or S;

Y denotes a halogen atom, an alkyl radical having at most six carbon atoms, a haloalkyl radical having at most six carbon atoms, an alkoxy radical having at most six carbon atoms, a haloalkoxy radical having at most six carbon atoms, an alkylthio radical having at most six carbon atoms, a haloalkylthio radical having at most six carbon atoms, an alkoxycarbonyl radical having at most six carbon atoms, a haloalkoxycarbonyl radical having at most six carbon atoms, an alkanoyloxy radical having at most six carbon atoms, a haloalkanoyloxy radical having at most six carbon atoms, an aryl radical, an aralkyl radical, a cyclohexyl radical containing 3 to 7 carbon atoms, or a cyano or nitro or hydroxyl radical;

n = positive integer less than 6, or zero, it being possible for the groups Y to be identical or different when n is larger than 1;

Z denotes an alkyl or alkoxy radical having at most six carbon atoms;

m = 0, 1 or 2, the groups Z being identical or different when m = 2;

and the salts of these compounds which are acceptable in agriculture.

**2.** Fungicidal composition according to claim 1, characterized in that R denotes an alkyl radical having at most six carbon atoms, optionally substituted with one or more substituents chosen from halogen atoms, an alkoxy radical having at most six carbon atoms and a radical $-Si(CH_2)_xR_1R_2R_3$, x = 0 or 1 and $R_1$, $R_2$ and $R_3$, which may be identical or different, being alkyl having at most six carbon atoms, alkoxy having at most six carbon atoms, aryl or aralkyl;

- an alkenyl radical, optionally substituted as when R denotes alkyl or with a $C_1$ to $C_4$ alkyl group;

A denotes a linear alkylene radical containing at most 4 carbon atoms;

X = 0;

n = 1, 2 or 3;

Y = haloalkoxy containing 1 to 3 carbon atoms, haloalkyl containing 1 to 3 carbon atoms, or halogen;

m = 0.

**3.** Fungicidal composition containing one or more compounds of formula (II),

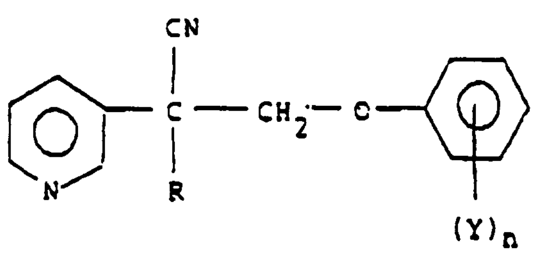

(II)

characterized in that R, n and Y have the same meaning as in claim 1 and, preferably, R denotes an alkyl radical having at most six carbon atoms, optionally substituted with one or more substituents chosen from halogen atoms, an alkoxy radical having at most six carbon atoms and a radical $-Si(CH_2)_xR_1R_2R_3$, x = 0 or 1 and $R_1$, $R_2$ and $R_3$, which may be identical or different, being alkyl having at most six carbon atoms, alkoxy having at most six carbon atoms, aryl or aralkyl, or an alkenyl radical, optionally substituted as when R denotes alkyl or with a $C_1$ to $C_3$ alkyl group, Y is haloalkoxy containing 1 to 3 carbon atoms, haloalkyl containing 1 to 3 carbon atoms, or halogen, n = 1, 2 or 3, it being possible for Y to be identical or different when n = 2 or 3,
and the salts of these compounds which are acceptable in agriculture.

**4.** Fungicidal composition according to claim 1, characterized in that n = 1 or 2 and in that Y is a halogen atom.

**5.** Fungicidal composition according to claim 1, characterized in that n = 1 or 2.

**6.** Fungicidal composition according to claim 1, characterized in that Y is at the 3- and/or 4-position(s).

**7.** Fungicidal composition according to claim 1, characterized in that R is a $C_1$-$C_6$ alkyl radical.

**8.** Process for treating crops attacked, or likely to be attacked, by fungal diseases, characterized in that an effective amount of a composition according to one of claims 1 to 7 is applied on the leaves.

**9.** Treatment process according to claim 8, characterized in that a dose of between 0.005 and 5 kg/ha of compound of formula (I) is applied.

**10.** Process for preparing the compounds of formula (I) in the case where A is methylene, characterized in that, in a first stage, a compound of formula (III) in which Z, R and m have the same meaning as in claim 1 is reacted with a dihalomethylene of formula (IV), Hal denoting a halogen atom, in a basic medium in a mole ratio III/IV of between 0.8 and 1.2, optionally in the presence of an inert solvent, leading to the compound of formula (V).
Then, in a second stage, the process consists in reacting the compound of formula (V) with the compound of formula (VI) in which X, Y and n have the same meaning as in formula (I), in the presence of a strong base such as alkali metal hydrides or alkali metal alcoholates, optionally in the presence of a suitable polar aprotic solvent.

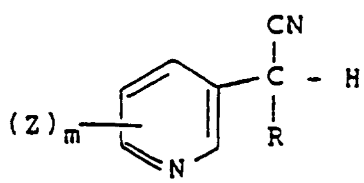

(III)

$(Hal)_2\ CH_2$

(IV)

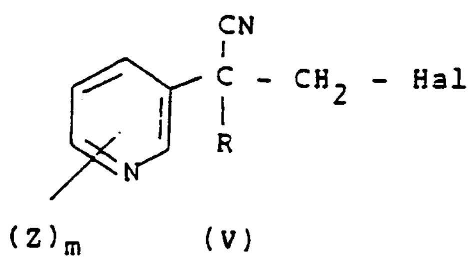

(V)

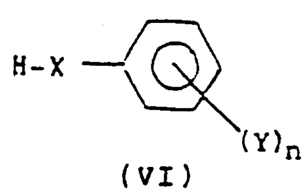

(VI)

**11.** Process for preparing the compounds of formula (I) in the case where A is other than methylene, characterized in that the process consists, in a first stage, in reacting the compound of formula (IX) with a compound of formula (VII) where A is other than methylene and Hal corresponds to a halogen atom, in the presence of a strong base, in a mole ratio (VII)/(IX) of between 0.8 and 1.2, optionally in the presence of a polar aprotic solvent, leading to the compound of formula (VIII); then, in a second stage, in reacting the compound of formula (VIII) with a compound of formula Hal-R, Hal being a halogen atom and R having the same meaning as in claim 1 except for H.

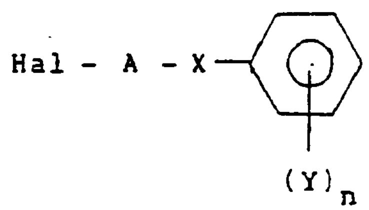

(VII)

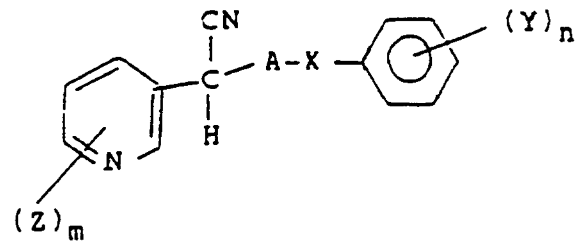

(VIII)

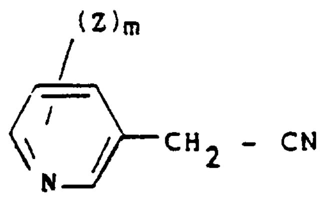

(IX)

**Patentansprüche**

**Patentansprüche für folgende Vertragsstaaten : BE, CH, DE, FR, GB, IT, LI, LU, NL, SE**

**1.** Verbindung der Formel I:

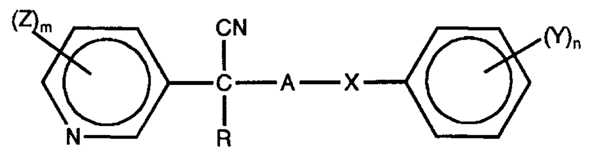

(I)

in der

A eine lineare Alkylenkette mit höchstens 6 Kohlenstoffatomen oder eine verzweigte Alkylenkette mit höchstens 6 Kohlenstoffatomen darstellt, unter der Bedingung, daß die lineare Kette von Methylen verschieden ist,

R

- einen Alkylrest mit höchstens 6 Kohlenstoffatomen darstellt, der gegebenenfalls durch einen oder mehrere Substituenten, ausgewählt aus den Halogenatomen und den Alkoxyresten mit höchstens 6 Kohlenstoffatomen, Alkylthioresten mit höchstens 6 Kohlenstoffatomen, $-Si(CH_2)_xR_1R_2R_3$, wobei x = 0, 1, und $R_1$, $R_2$ und $R_3$, identisch oder verschieden, Alkylreste mit höchstens 6 Kohlenstoffatomen, Alkoxyreste mit höchstens 6 Kohlenstoffatomen, Aryl- oder Aralkylreste sind, substituiert ist;
- einen Cycloalkylrest mit 3 bis 7 Kettengliedern darstellt, der gegebenenfalls selbst substituiert ist, wie der Alkylrest;
- einen Aralkylrest darstellt, der gegebenenfalls in gleicher Weise wie der Alkylrest substituiert ist;
- einen mono- oder mehrfach ungesättigten Alkinyl- oder Alkenylrest mit höchstens 6 Kohlenstoffatomen, der gegebenenfalls in gleicher Weise substituiert ist, wie der Alkylrest, unter der Bedingung, daß keine ungesättigte Bindung in $\alpha$-Stellung zu dem Kohlenstoffatom steht, das die CN-Gruppe trägt;

wobei außerdem R ein Wasserstoffatom sein kann, wenn A verschieden von Methylen ist.

X = O oder S ist
Y ein Halogenatom, einen Alkylrest mit höchstens 6 Kohlenstoffatomen, einen Haloalkylrest mit höchstens 6 Kohlenstoffatomen, einen Alkoxyrest mit höchstens 6 Kohlenstoffatomen, einen Haloalkoxyrest mit höchstens 6 Kohlenstoffatomen, einen Alkylthiorest mit höchstens 6 Kohlenstoffatomen, einen Haloalkylthiorest mit höchstens 6 Kohlenstoffatomen, einen Alkoxycarbonylrest mit höchstens 6 Kohlenstoffatomen, einen Haloalkoxycarbonylrest mit höchstens 6 Kohlenstoffatomen, einen Alkanoyloxyrest mit höchstens 6 Kohlenstoffatomen, einen Haloalkanoyloxyrest mit höchstens 6 Kohlenstoffatomen, einen Arylrest, einen Aralkylrest, einen Cyclohexylrest mit 3 bis 7 Kohlenstoffatomen, einen Cyano- oder Nitro- oder Hydroxylrest darstellt.
n eine ganze positive Zahl oder 0 darstellt, die kleiner als 6 ist, wobei die Reste Y identisch oder verschieden sein können, wenn n größer ist als 1;
Z einen Alkylrest mit höchstens 6 Kohlenstoffatomen oder einen Alkoxyrest mit höchstens 6 Kohlenstoffatomen darstellt;
m = 0, 1, 2 ist, wobei die Reste Z identisch oder verschieden sind, wenn m = 2 ist;
sowie die für die Landwirtschaft verträglichen Salze dieser Verbindungen.

**2.** Verbindung der Formel (I) nach Anspruch 1, dadurch gekennzeichnet, daß R einen Alkylrest mit höchstens 6 Kohlenstoffatomen darstellt, der gegebenenfalls durch einen oder mehrere Substituenten substituiert ist, ausgewählt aus den Halogenatomen, einem Alkoxyrest mit höchstens 6 Kohlenstoffatomen, einem Rest $-Si(CH_2)_xR_1R_2R_3$, wobei x = 0 oder 1 ist und $R_1$, $R_2$ und $R_3$, identisch oder verschieden, einen Alkylrest mit höchstens 6 Kohlenstoffatomen, einen Alkoxyrest mit höchstens 6 Kohlenstoffatomen, einen Arylrest oder einen Aralkylrest darstellen;
R einen Alkenylrest darstellt, der gegebenenfalls durch eine $C_1$- bis $C_4$-Alkylgruppe oder in gleicher Weise substituiert ist, wie wenn R einen Alkylrest darstellt;
A einen linearen Alkylenrest darstellt, der bis zu 4 Kohlenstoffatome enthält;
X = 0 ist
n = 1, 2 oder 3 ist
Y einen Haloalkoxyrest mit 1 bis 3 Kohlenstoffatomen, einen Haloalkylrest mit 1 bis 3 Kohlenstoffatomen oder ein Halogen darstellt
m = 0 ist.

**3.** Verbindung der Formel (II)

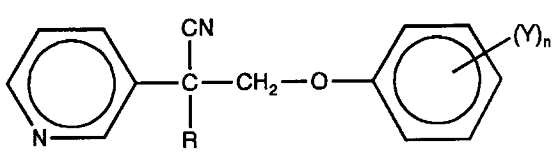

(II)

dadurch gekennzeichnet, daß R, n und Y die gleiche Bedeutung wie in dem Anspruch 1 haben und vorzugsweise R einen Alkylrest mit höchstens 6 Kohlenstoffatomen darstellt, der gegebenenfalls durch einen oder mehrere Substituenten substituiert ist, ausgewählt aus den Halogenatomen, einem Alkoxyrest mit höchstens 6 Kohlenstoffatomen, einem Rest $-Si(CH_2)_xR_1R_2R_3$, wobei x = 0, 1 und $R_1$, $R_2$ und $R_3$, identisch oder verschieden, einen Alkylrest mit höchstens 6 Kohlenstoffatomen, einen Alkoxyrest mit höchstens 6 Kohlenstoffatomen, einen Aryl- oder einen Aralkylrest darstellen, einem Alkenylrest, der gegebenenfalls durch eine $C_1$- bis $C_3$-Alkylgruppe oder in gleicher Weise substituiert ist, als wenn R einen Alkylrest darstellt, Y ein Haloalkoxyrest mit 1 bis 3 Kohlenstoffatomen, ein Haloalkylrest mit 1 bis 3 Kohlenstoffatomen oder ein Halogenatom ist,
n = 1, 2 oder 3 ist
Y identisch oder verschieden sein kann, wenn n = 2 oder 3 ist
sowie die für die Landwirtschaft verträglichen Salze dieser Verbindungen.

4. Verbindung nach Anspruch 1, dadurch gekennzeichnet, daß n = 1 oder 2 ist und daß Y ein Halogenatom ist.

5. Verbindung nach Anspruch 1, dadurch gekennzeichnet, daß n = 1 oder 2 ist.

6. Verbindung nach Anspruch 1, dadurch gekennzeichnet, daß Y in 3- und/oder 4-Stellung steht.

7. Verbindung nach Anspruch 1, dadurch gekennzeichnet, daß R ein $C_1$-bis $C_6$-Alkylrest ist.

8. Verwendung der Verbindungen nach einem der Ansprüche 1 bis 7 als Fungizid, insbesondere in Form fungizider Zusammensetzungen, enthaltend 0,5 bis 95 % der Verbindungen in Kombination mit festen oder flüssigen, für die Landwirtschaft verträglichen Trägern und/oder oberflächenaktiven, ebenfalls für die Landwirtschaft verträglichen Mitteln.

9. Verfahren zur Behandlung von Kulturen, die durch Pilzkrankheiten befallen sind oder befallen werden können, dadurch gekennzeichnet, daß man auf die Blätter eine wirksame Menge einer Verbindung gemäß einem der Ansprüche 1 bis 7 aufbringt.

10. Verfahren zur Behandlung nach Anspruch 9, dadurch gekennzeichnet, daß man eine Dosis zwischen 0,005 und 5 kg/ha aufbringt.

11. Als Zwischenprodukte bei der Herstellung der Verbindungen nach den Ansprüchen 1 bis 7 verwendbare Verbindungen, die den Formeln (V, VIII) entsprechen:

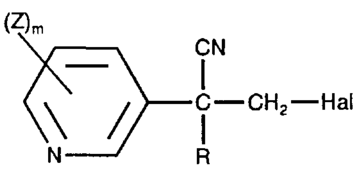

(V)

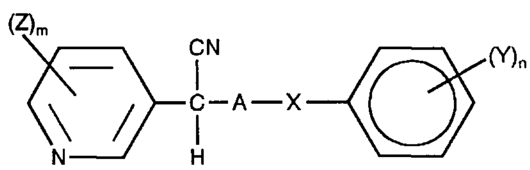

(VIII)

12. Verfahren zur Herstellung von Verbindungen der Formel (I), bei denen A Methylen ist, dadurch gekennzeichnet, daß man in einem ersten Schritt eine Verbindung der Formel (III), in der Z, R und m die gleiche Bedeutung haben wie in dem Anspruch 1, mit einem Dihalogenmethylen der Formel (IV), in der Hal ein Halogenatom darstellt, in basischem Milieu in einem molaren Verhältnis von III : IV zwischen 0,8 und 1,2, gegebenenfalls in Gegenwart eines inerten Lösungsmittels reagieren läßt, so daß man eine Verbindung der Formel (V) erhält; und dann in einem zweiten Schritt die Verbindung der Formel (V) mit der Verbindung der Formel (VI), in der X, Y und n die gleiche Bedeutung haben wie in Formel (I), in Gegenwart einer starken Base, insbesondere eines Alkalimetallhydrids oder Alkalimetallalkoholats, gegebenenfalls in Gegenwart eines geeigneten polaren aprotischen Lösungsmittels reagieren läßt.

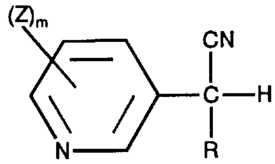

(III)

$(Hal)_2CH_2$

(IV)

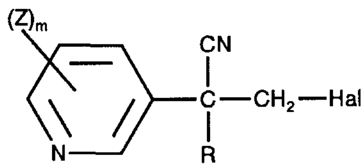

(V)

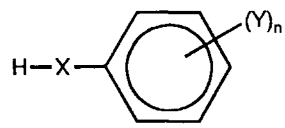

(VI)

**13.** Verfahren zur Herstellung einer Verbindung der Formel (I), in der A nicht Methylen ist, dadurch gekennzeichnet, daß das Verfahren darin besteht, in einem ersten Schritt die Verbindung der Formel (IX) mit einer Verbindung der Formel (VII), in der A nicht Methylen ist und Hal ein Halogenatom darstellt, in Gegenwart einer starken Base in einem molaren Verhältnis von (VIII) : (IX) zwischen 0,8 und 1,2, gegebenenfalls in Gegenwart eines polaren aprotischen Lösemittels reagieren läßt, wobei man eine Verbindung der Formel (VIII) erhält; und dann in einem zweiten Schritt die Verbindung der Formel (VIII) mit einer Verbindung der Formel Hal-R reagieren läßt, wobei Hal ein Halogenatom ist und R die gleiche Bedeutung hat wie in dem Anspruch 1, jedoch nicht H ist.

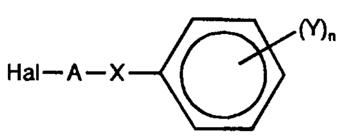

(VI)

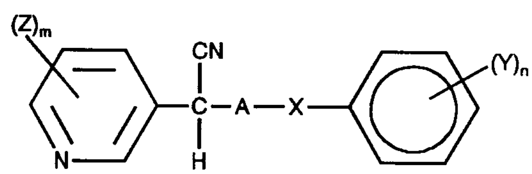

(VIII)

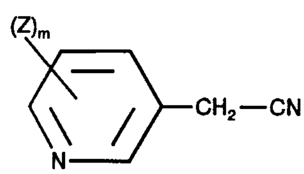

(IX)

**14.** Vermehrungsprodukt für Kulturpflanzen, das einer Schutzbehandlung mit einer der Verbindung nach einem der Ansprüche 1 bis 7 unterzogen wurde.

**15.** Samenkorn, das einer Schutzbehandlung mit einer Verbindung nach einem der Ansprüche 1 bis 7 unterzogen wurde.

**Patentansprüche für folgende Vertragsstaaten : AT, ES, GR**

**1.** Fungizide Zusammensetzung, enthaltend eine oder mehrere Verbindungen der Formel I:

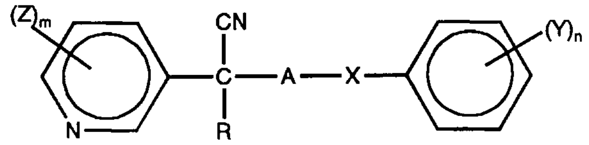

(I)

in der

A eine lineare Alkylenkette mit höchstens 6 Kohlenstoffatomen oder eine verzweigte Alkylenkette mit höchstens 6 Kohlenstoffatomen darstellt, unter der Bedingung, daß die lineare Kette von Methylen verschieden ist,

R

- einen Alkylrest mit höchstens 6 Kohlenstoffatomen darstellt, der gegebenenfalls durch einen oder mehrere Substituenten, ausgewählt aus den Halogenatomen und den Alkoxyresten mit höchstens 6 Kohlenstoffatomen, Alkylthioresten mit höchstens 6 Kohlenstoffatomen, $-Si(CH_2)_xR_1R_2R_3$, wobei x = 0, 1, und $R_1$, $R_2$ und $R_3$, identisch oder verschieden, Alkylreste mit höchstens 6 Kohlenstoffatomen, Alkoxyreste mit höchstens 6 Kohlenstoffatomen, Aryl- oder Aralkylreste sind, substituiert ist;
- einen Cycloalkylrest mit 3 bis 7 Kettengliedern darstellt, der gegebenenfalls selbst substituiert ist, wie der Alkylrest;
- einen Aralkylrest darstellt, der gegebenenfalls in gleicher Weise wie der Alkylrest substituiert ist;
- einen mono- oder mehrfach ungesättigten Alkinyl- oder Alkenylrest mit höchstens 6 Kohlenstoffatomen, der gegebenenfalls in gleicher Weise substituiert ist, wie der Alkylrest, unter der Bedingung, daß keine ungesättigte Bindung in α-Stellung zu dem Kohlenstoffatom steht, das die CN-Gruppe trägt;

  wobei außerdem R ein Wasserstoffatom sein kann, wenn A verschieden von Methylen ist.

X = O oder S ist

Y ein Halogenatom, einen Alkylrest mit höchstens 6 Kohlenstoffatomen, einen Haloalkylrest mit höchstens 6 Kohlenstoffatomen, einen Alkoxyrest mit höchstens 6 Kohlenstoffatomen, einen Haloalkoxyrest mit höchstens 6 Kohlenstoffatomen, einen Alkylthiorest mit höchstens 6 Kohlenstoffatomen, einen Haloalkylthiorest mit höchstens 6 Kohlenstoffatomen, einen Alkoxycarbonylrest mit höchstens 6 Kohlenstoffatomen, einen Haloalkoxycarbonylrest mit höchstens 6 Kohlenstoffatomen, einen Alkanoyloxyrest mit höchstens 6 Kohlenstoffatomen, einen Haloalkanoyloxyrest mit höchstens 6 Kohlenstoffatomen, einen Arylrest, einen Aralkylrest, einen Cyclohexylrest mit 3 bis 7 Kohlenstoffatomen, einen Cyano- oder Nitro- oder Hydroxylrest darstellt.

n eine ganze positive Zahl oder 0 darstellt, die kleiner als 6 ist, wobei die Reste Y identisch oder verschieden sein können, wenn n größer ist als 1;

Z einen Alkylrest mit höchstens 6 Kohlenstoffatomen oder einen Alkoxyrest mit höchstens 6 Kohlenstoffatomen darstellt;

m = 0, 1, 2 ist, wobei die Reste Z identisch oder verschieden sind, wenn m = 2 ist;

sowie die für die Landwirtschaft verträglichen Salze dieser Verbindungen.

**2.** Fungizide Zusammensetzung nach Anspruch 1, dadurch gekennzeichnet, daß R einen Alkylrest mit höchstens 6 Kohlenstoffatomen darstellt, der gegebenenfalls durch einen oder mehrere Substituenten substituiert ist, ausgewählt aus den Halogenatomen, einem Alkoxyrest mit höchstens 6 Kohlenstoffatomen, einem Rest $-Si(CH_2)_xR_1R_2R_3$, wobei x = 0 oder 1 ist und $R_1$, $R_2$ und $R_3$, identisch oder verschieden, einen Alkylrest mit höchstens 6 Kohlenstoffatomen, einen Alkoxyrest mit höchstens 6 Kohlenstoffatomen, einen Arylrest oder einen Aralkylrest darstellen;

R einen Alkenylrest darstellt, der gegebenenfalls durch eine $C_1$- bis $C_4$-Alkylgruppe oder in gleicher Weise substituiert ist, wie wenn R einen Alkylrest darstellt;
A einen linearen Alkylenrest darstellt, der bis zu 4 Kohlenstoffatome enthält;
X = 0 ist
n = 1, 2 oder 3 ist
Y einen Haloalkoxyrest mit 1 bis 3 Kohlenstoffatomen, einen Haloalkylrest mit 1 bis 3 Kohlenstoffatomen oder ein Halogen darstellt
m = 0 ist.

**3.** Fungizide Zusammensetzung, enthaltend eine oder mehrere Verbindungen der Formel (II)

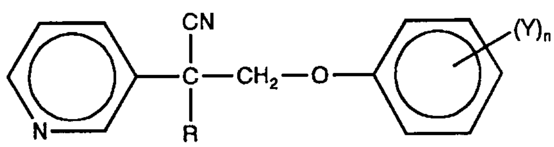

(II)

dadurch gekennzeichnet, daß R, n und Y die gleiche Bedeutung wie in dem Anspruch 1 haben und vorzugsweise R einen Alkylrest mit höchstens 6 Kohlenstoffatomen darstellt, der gegebenenfalls durch einen oder mehrere Substituenten substituiert ist, ausgewählt aus den Halogenatomen, einem Alkoxyrest mit höchstens 6 Kohlenstoffatomen, einem Rest $-Si(CH_2)_xR_1R_2R_3$, wobei x = 0, 1 und $R_1$, $R_2$ und $R_3$, identisch oder verschieden, einen Alkylrest mit höchstens 6 Kohlenstoffatomen, einen Alkoxyrest mit höchstens 6 Kohlenstoffatomen, einen Aryl- oder einen Aralkylrest darstellen, einem Alkenylrest, der gegebenenfalls durch eine $C_1$- bis $C_3$-Alkylgruppe oder in gleicher Weise substituiert ist, als wenn R einen Alkylrest darstellt, Y ein Haloalkoxyrest mit 1 bis 3 Kohlenstoffatomen, ein Haloalkylrest mit 1 bis 3 Kohlenstoffatomen oder ein Halogenatom ist,
n = 1, 2 oder 3 ist
Y identisch oder verschieden sein kann, wenn n = 2 oder 3 ist
sowie die für die Landwirtschaft verträglichen Salze dieser Verbindungen.

**4.** Fungizide Zusammensetzung nach Anspruch 1, dadurch gekennzeichnet, daß n = 1 oder 2 ist und daß Y ein Halogenatom ist.

**5.** Fungizide Zusammensetzung nach Anspruch 1, dadurch gekennzeichnet, daß n = 1 oder 2 ist.

**6.** Fungizide Zusammensetzung nach Anspruch 1, dadurch gekennzeichnet, daß Y in 3- und/oder 4-Stellung steht.

**7.** Fungizide Zusammensetzung nach Anspruch 1, dadurch gekennzeichnet, daß R ein $C_1$- bis $C_6$-Alkylrest ist.

**8.** Verfahren zur Behandlung von Kulturen, die durch Pilzkrankheiten befallen sind oder befallen werden können, dadurch gekennzeichnet, daß man auf die Blätter eine wirksame Menge einer Verbindung gemäß einem der Ansprüche 1 bis 7 aufbringt.

**9.** Verfahren zur Behandlung nach Anspruch 8, dadurch gekennzeichnet, daß man eine Dosis der Verbindung der Formel (I) zwischen 0,005 und 5 kg/ha aufbringt.

**10.** Verfahren zur Herstellung von Verbindungen der Formel (I), bei denen A Methylen ist, dadurch gekennzeichnet, daß man in einem ersten Schritt eine Verbindung der Formel (III), in der Z, R und m die gleiche Bedeutung haben wie in dem Anspruch 1, mit einem Dihalogenmethylen der Formel (IV), in der Hal ein Halogenatom darstellt, in basischem Milieu in einem molaren Verhältnis von III : IV zwischen 0,8 und 1,2, gegebenenfalls in Gegenwart eines inerten Lösungsmittels reagieren läßt, so daß man eine Verbindung der Formel (V) erhält; und dann in einem zweiten Schritt die Verbindung der Formel (V) mit der Verbindung der Formel (VI), in der X, Y und n die gleiche Bedeutung haben wie in Formel (I), in Gegenwart einer starken Base, insbesondere eines Alkalimetallhydrids oder Alkalimetallalkoholats, gegebenenfalls in Gegenwart eines geeigneten polaren aprotischen Lösungsmittels reagieren

läßt.

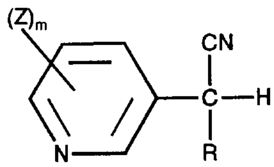

(III)

$(Hal)_2CH_2$

(IV)

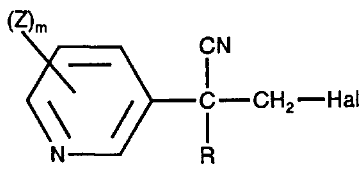

(V)

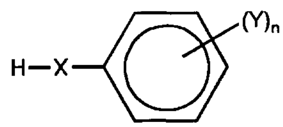

(VI)

**11.** Verfahren zur Herstellung einer Verbindung der Formel (I), in der A nicht Methylen ist, dadurch gekennzeichnet, daß das Verfahren darin besteht, in einem ersten Schritt die Verbindung der Formel (IX) mit einer Verbindung der Formel (VII), in der A nicht Methylen ist und Hal ein Halogenatom darstellt, in Gegenwart einer starken Base in einem molaren Verhältnis von (VIII) : (IX) zwischen 0,8 und 1,2, gegebenenfalls in Gegenwart eines polaren aprotischen Lösemittels reagieren läßt, wobei man eine Verbindung der Formel (VIII) erhält; und dann in einem zweiten Schritt die Verbindung der Formel (VIII) mit einer Verbindung der Formel Hal-R reagieren läßt, wobei Hal ein Halogenatom ist und R die gleiche Bedeutung hat wie in dem Anspruch 1, jedoch nicht H ist.

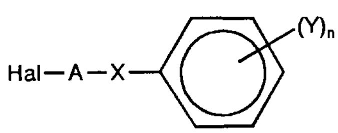

(VI)

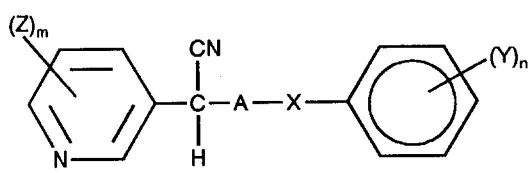

(VIII)

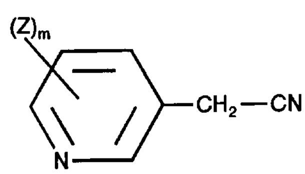

(IX)